(19) Europäisches Patentamt
European Patent Office
Office européen des brevets

(11) Veröffentlichungsnummer: **0 199 985**
**A2**

# (12) EUROPÄISCHE PATENTANMELDUNG

(21) Anmeldenummer: **86104007.9**

(22) Anmeldetag: **24.03.86**

(51) Int. Cl.[4]: **C07C 65/19** , C07C 69/773 , C07C 143/24 , C07F 7/02 , C07C 49/21 , C07D 309/02 , C07C 103/60 , C07C 83/08 , //A01K31/19

(30) Priorität: **27.04.85 DE 3515278**

(43) Veröffentlichungstag der Anmeldung:
**10.12.86 Patentblatt 86/45**

(84) Benannte Vertragsstaaten:
**AT BE CH DE FR GB IT LI LU NL SE**

(71) Anmelder: **Grünenthal GmbH**
**Patentabteilung Steinfeldstrasse 2**
**D-5190 Stolberg(DE)**

(72) Erfinder: **Vollenberg, Werner, Dr.**
**Eupenerstrasse 26a**
**D-5190 Stolberg(DE)**
Erfinder: **Zimmer, Oswald, Dr.**
**Weckspfad 4**
**D-5160 Düren(DE)**
Erfinder: **Loschen, Gerriet, Dr.**
**Erzweg 4**
**D-5190 Stolberg(DE)**
Erfinder: **Kiesewetter, Erwin, Dr.**
**Obere Donnerbergstrasse 57**
**D-5190 Stolberg(DE)**
Erfinder: **Seipp, Ulrich, Dr.**
**Pfeilstrasse 5**
**D-5100 Aachen(DE)**

## (54) Neue Benzoesäurederivate, diese enthaltende Arzneimittel und Verfahren zur Herstellung dieser Verbindungen und Arzneimittel.

(57) Die erfindungsgemäßen neuen Benzoesäurederivate sind spezifisch wirkende, hinreichend stabile 5-Lipoxygenase-Inhibitoren und insbesondere in Arzneimittelzubereitungsformen zur Therapie pathologischer Zustände, an denen Leukotriene als Mediatoren beteiligt sind, einsetzbar. Sie entsprechen der allgemeinen Formel

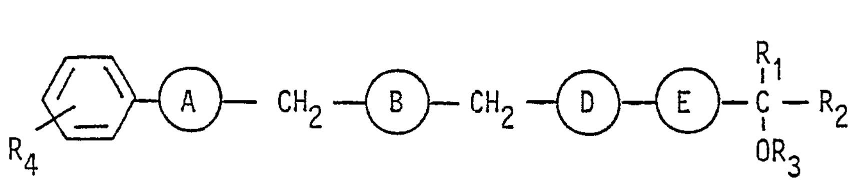

I

worin A und E für -C≡C-und B und D für $-CH_2-CH_2-$ stehen oder A, B und D jeweils für -C≡C-oder cis-CH = CH-und E für trans-CH = CH-stehen,

$R_1$ Wasserstoff, Methyl oder Ethyl ist, $R_2$ Wasserstoff, ein gegebenenfalls wie definiert substituierter Alkyl-oder Phenylrest oder ein Cycloalkylrest ist, $R_3$ Wasserstoff, Acetyl oder Propionyl darstellt und $R_4$



Rank Xerox

für eine Carboxylgruppe oder ein funktionelles Derivat davon, wie ein Salz, einen Ester, ein Amid oder eine Hydroxamsäure, oder für eine Nitrilgruppe steht.

Die erfindungsgemäße Herstellung der Verbindungen der Formel I erfolgt insbesondere durch Aufbau der aliphatischen Kette ausgehend von geeigneten Acetylenverbindungen. Der Rest $R_4$ kann anschließend in andere gewünschte Carboxylgruppenderivate umgewandelt werden. Durch teilweise oder vollständige Hydrierung lassen sich A, B, D und E im Rahmen der Definition variieren.

# Neue Benzoesäurederivate, diese enthaltende Arzneimittel und Verfahren zur Herstellung dieser Verbindungen und Arzneimittel.

Polyungesättigte höhere Fettsäuren wie z. B. die Arachidonsäure dienen im Stoffwechsel des Menschen und von Säugetieren als Substrate für die enzymatisch katalysierte Bildung physiologisch bedeutsamer Eicosanoide wie z. B. Prostaglandine und Leukotriene, einer auch unter dem Namen "Slow reacting Substance of Anaphylaxis" (SRS-A) bekannten Substanzklasse. Dabei wird die Prostaglandinbildung durch die Cyclo-oxygenase (auch als "Prostaglandinsynthetase" bezeichnet), die Leukotrienbildung durch 5-Lipoxygenasen katalysiert.

Während die Prostaglandine eine Anzahl erwünschter Wirkungen im Organismus entfalten, ist von den Leukotrienen bzw. der SRS-A bekannt, daß sie für die Entstehung von allergischen Reaktionen, Bronchokonstriktionen, Entzündungen, Asthma und eine Vielzahl weiterer unerwünschter Effekte verantwortlich sind. Es ist daher wünschenswert, über chemisch und metabolisch stabile Verbindungen verfügen zu können, die im Organismus die Prostaglandinbildung unbeeinflußt lassen, gleichzeitig aber möglichst selektiv bzw. spezifisch die 5-Lipoxygenase hemmen und so die Bildung der unerwünschten Leukotriene verhindern. Es wurde nunmehr gefunden, daß bestimmte neue Benzoesäurederivate chemisch und metabolisch für eine therapeutische Anwendung hinreichend stabil sind und eine spezifische Hemmwirkung gegenüber der 5-Lipoxygenase besitzen.

Diese neuen Benzoesäurederivate entsprechen der allgemeinen Formel

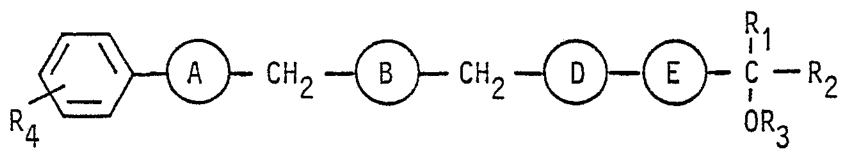

I

worin

A und E für -C≡C-und B und D für $CH_2$-$CH_2$-stehen oderA, B und D jeweils für -C≡C-oder cis-CH=CH- und E für trans-CH=CH-stehen,

$R_1$ Wasserstoff, Methyl oder Ethyl ist,

$R_2$ Wasserstoff oder

einen geradkettigen Alkylrest mit 1 bis 6 Kohlenstoffatomen oder eine 5-bis 7-gliedrige Cycloalkylgruppe, vorzugsweise die Cyclohexylgruppe, oder

eine Gruppe der Formel $-(CH_2)_m-O-R_5$, worin m für eine der Zahlen 1, 2 oder 3 und $R_5$ für Methyl oder Ethyl steht, oder

eine Gruppe der Formel

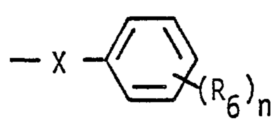

worin X für eine einfache Bindung, eine $-CH_2$-Gruppe oder für eine $-CH_2O$-Gruppe steht und $R_6$ ein Wasserstoff-, Fluor-oder Chloratom oder eine Methyl-, Methoxy-oder Trifluormethylgruppe bedeutet und n für eine der Zahlen 1 oder 2 steht,

bedeutet,

$R_3$ Wasserstoff oder ein Acetyl-oder Propionylrest ist

und

$R_4$ für eine Gruppe der Formel $-COOR_7$, worin $R_7$ ein Wasserstoffatom, ein pharmazeutisch verträgliches, insbesondere ein einwertiges Kation, ein geradkettiger oder verzweigter Alkylrest mit 1 bis 6 Kohlenstoffatomen oder die Gruppe $-(CH_2)_2-N[-(CH_2)-CH_3]_2$ ist, in der p für Null oder eine der Zahlen 1 bis 3 steht, sowie pharmazeutisch verträgliche Salze dieser basischen Ester mit Säuren, oder

für eine Gruppe der Formel

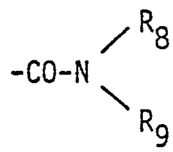

worin $R_8$ und $R_9$ gleich oder verschieden sind und für Wasserstoff, einen geradkettigen oder verzweigten Alkylrest mit 1 bis 6 Kohlenstoffatomen oder für 2-Hydroxyethyl stehen oder einer dieser Reste eine Hydroxylgruppe, der andere Wasserstoff bedeutet,

oder $R_8$ und $R_9$ zusammengenommen die Gruppe -$(CH_2)_q$-darstellen, in der q für eine der Zahlen 4, 5 oder 6 steht, oder

für eine Gruppe der Formel

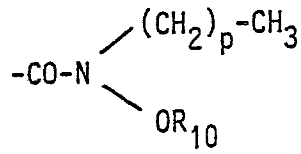

worin $R_{10}$ für Wasserstoff, -$(CH_2)_p$-$CH_3$, Carboxymethyl, Acetyl oder Propionyl steht und p jeweils die gleiche Bedeutung wie oben hat, oder

für eine Gruppe der Formel -CO-NH-$(CH_2)_r$-$N(CH_3)_2$,

worin r für eine der Zahlen 2 oder 3 steht, und deren pharmazeutisch verträgliche Salze mit Säuren, oder

für eine Gruppe der Formel

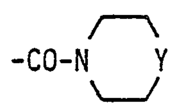

in der Y ein Sauerstoffatom oder die Gruppe

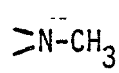

bedeutet und gegebenenfalls deren pharmazeutisch verträgliche Salze mit Säuren,

oder für einen Nitrilrest

steht.

Je nach der Bedeutung der Gruppen A, B, D und E lassen sich die Verbindungen der Formel I durch folgende Formelbilder wiedergeben:

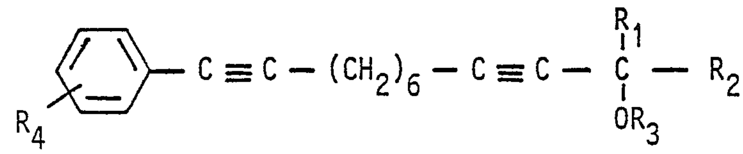

I'

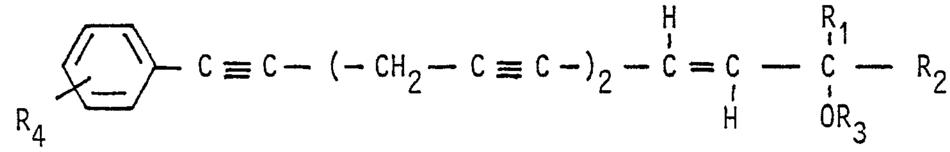

I''

oder

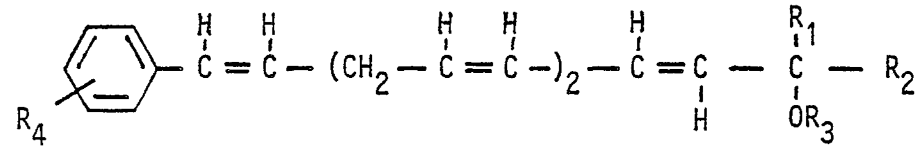

I'''

worin jeweils $R_1$ bis $R_4$ die gleiche Bedeutung wie oben haben.

Besonders bevorzugt sind von diesen Verbindungen diejenigen der Formel I', d. h. Verbindungen der Formel I, in denen A und E für -C≡C- sowie B und D für $-CH_2-CH_2-$ stehen.

In bevorzugten Verbindungen der Formel I stehen $R_1$ und $R_3$ für Wasserstoff, während $R_2$ darin vorzugsweise Wasserstoff, einen Alkylrest mit 5 oder 6 Kohlenstoffatomen oder einen Cyclohexylrest bedeutet.

Der Rest $R_4$ steht in den Verbindungen der Formel I bevorzugt für eine Carboxylgruppe, gegebenenfalls in Form des Natrium-oder Kaliumsalzes, für eine Gruppe der Formel $-COO-(CH_2)_p-CH_3$, worin p wie oben definiert ist, oder für die Gruppe der obigen Formel

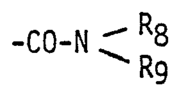

. Ganz besonders bevorzugt sind aber die Verbindungen der Formel I, in denen $R_4$ die Gruppe der Formel

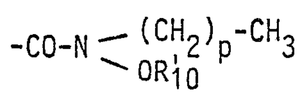

verkörpert, worin $R'_{10}$ für Wasserstoff, $-(CH_2)_p-CH_3$ oder Acetyl steht und p jeweils die gleiche Bedeutung wie oben hat

Einzelvertreter der bevorzugten Verbindungen sind z. B.

3-(11'-Hydroxy-undeca-1',9'-diin-1'-yl)-benzoesäureethylester (Produkt des Beispiels 18)

3-(11'-Hydroxy-undeca-1',9'-diin-1'-yl)-benzoesäurepiperidid (Produkt des Beispiels 17),

3-(11'-Hydroxy-undeca-1',9'-diin-1'-yl-N-methyl-benzhydroxamsäure (Produkt des Beispiels 16),

4-(11'-Hydroxy-undeca-1',9'-diin-1'-yl)-N-methylbenzhydroxamsäure (Produkt des Beispiels 11),

3-(11'-Hydroxy-hexadec-9'E-en-1',4',7'-triin-1'-yl)-benzoesäuremethylester (Produkt des Beispiels 1),

3-(11'-Cyclohexyl-11'-hydroxy-undec-9'E-en-

1',4',7'-triin-1'-yl)-benzoesäuremethylester (Produkt des Beispiels 4),

4-(11'-Hydroxy-hexadeca-1'Z,4'Z,7'Z,9'E-tetraen-1'-yl)-benzoesäuremethylester (Produkt des Beispiels 7),

3-(11'-Cyclohexyl-11'-hydroxy-undeca-1',9'-diin-1'-yl)-N-methyl-benzhydroxamsäure (Produkt des Beispiels 23biii),

3-(11'-Hydroxy-undeca-1',9',-diin-1'-yl)-benzoesäure-[N,N-di-(n-propyl)]-amid (Produkt des Beispiels 24g)

und weitere Verbindungen.

Wie bereits einleitend ausgeführt, besitzen die Verbindungen der Formel I eine spezifische Hemmwirkung gegenüber der 5-Lipoxygenase, was durch in-vitro-Experimente ermittelt wurde.

Zur Ermittlung der 5-Lipoxygenasehemmung wurden basophile leukämische Leukozyten der Ratte in-vitro gezüchtet und nach Erreichen einer Zelldichte von ca. $10^6$ Zellen pro ml bei 400 g vom Nährmedium abzentrifugiert. Der Rückstand wurde in 50 mM Kaliumphosphatpuffer vom pH 7,4 so suspendiert, daß die Zellzahl 1,5 • $10^7$ Zellen pro ml betrug.

Jeweils 1 ml dieser Suspension wurde mit Indomethacin (10 µM) und Calciumchlorid (2 mM) versetzt und in An-oder Abwesenheit einer der Testsubstanzen mit radioaktiv markierter Arachidonsäure und dem Calcium-Ionophor A 23 187 bei Raumtemperatur für 5 Minuten inkubiert. Nach Ansäuern auf pH 5 wurden die unter dem Einfluß der 5-Lipoxygenase gebildeten Arachidonsäuremetaboliten mit Ethylacetat extrahiert und mit einem für die Leukotriene geeigneten Fließmittelgemisch dünnschichtchromatographisch getrennt [vgl. Jakschik et al. Biochem. Biophys. Res. Commun. 102, 624 (1981)]. Die Radioaktivitätsverteilung auf die verschiedenen Metaboliten wurde mit Hilfe eines Dünnschichtscanners gemessen. Setzt man die prozentualen Bildungsanteile der 5-Lipoxygenaseprodukte (5-HETE und $LTB_4$) zu der Menge bzw. Konzentration der eingesetzten Gesamtradioaktivität bzw. der Testsubstanz der Formel I in Relation, so läßt sich der $IC_{50}$-Wert - (d. h. die Konzentration, die eine 50 %-ige Hemmung der 5-Lipoxygenase bewirkt) ermitteln.

So wurden beispielsweise für die oben aufgeführten sowie weitere in den Beispielen beschriebene Verbindungen folgende Werte gefunden:

| Produkt des Beispiels | $IC_{50}$ [µM] |
|---|---|
| 18 | 7,5 |
| 17 | 2,3 |
| 16 | 1,0 |
| 11 | 0,2 |
| 1 | 1,7 |
| 4 | 1,4 |

| Produkt des Beispiels | $IC_{50}$ [µM] |
|---|---|
| 7 | 1,0 |
| 23biii | 0,5 |
| 24g | 2,4 |
| 24l | 0,5 |
| 24n | 1,6 |
| 27a | 7,0 |

Der Einfluß der Testsubstanzen auf die Cyclooxygenaseaktivität wurde mit Hilfe von Schafssamenblasenmikrosomen, suspendiert in Kaliumphosphatpuffer (50 mM, pH 7,0) in Gegenwart von 2,2'-Azino-di-[3-ethylbenzothiazol in-sulfonsäure-(6)] durch Inkubation mit der Testsubstanz und Arachidonsäure und photometrische Messung bei 420 nm untersucht. Dabei zeigte sich, daß die $IC_{50}$-Werte für die Hemmung der Cyclooxygenase um mehr als das 50-fache, teilweise um mehr als das 400-fache höher als die $IC_{50}$-Werte für die 5-Lipoxygenasehemmung liegen, d. h. daß die Testsubstanzen sehr spezifisch nur die Hemmung der Lipoxygenaseaktivität bewirken.

Aufgrund dieses günstigen Einflusses auf die Metabolisierung polyungesättigter Fettsäuren, insbesondere der Hemmwirkung auf die Bildung von Arachidonsäuremetaboliten der 5-Lipoxygenase wie 5-Hydroxyperoxyeicosatetraensäure (5-HPETE), 5-Hydroxyeicosatetraensäure (5-HETE) bzw. SRS-A bewirken die erfindungsgemäßen Verbindungen der Formel I im Organismus des Menschen bzw. von Säugetieren zahlreiche wertvolle physiologische Effekte wie z. B. antiallergische, antianaphylaktische, antiphlogistische, antiasthmatische, blutdrucksenkende und durchblutungsfördernde (coronarer und cerebraler Kreislauf) Wirkungen, Verminderung der Leukozytenaggregation bzw. der Bildung von Leukozytenthromben usw. Da die erfindungsgemäßen Verbindungen der Formel I chemisch und metabolisch für eine therapeutische Anwendung hinreichend stabil und lagerfähig sind, eignen sie sich zum Einsatz als Arzneimittel z. B. als Antiallergica, Antianaphylaktica, Antiphlogistica, Antiasthmatica, Antihypertensiva, antithrombotische Mittel, Mittel zur Prophylaxe oder Therapie von ischämischem Herzinfarkt, Störungen der coronaren und/oder cerebralen Arterien usw.

Die erfindungsgemäßen Verbindungen besitzen nur eine geringe Toxizität, die sich erst bei weit höheren als den therapeutisch oder prophylaktisch anzuwendenden Dosierungen bemerkbar macht. Sie können daher als solche oder in geeigneten pharmazeutischen Zubereitungen an Mensch oder Tier verabreicht werden.

Die Erfindung betrifft dementsprechend auch Arzneimittel, die als Wirkstoff eine oder mehrere der erfindungsgemäßen Verbindungen der Formel I enthalten. Die an den Patienten zu verabreichende Wirkstoffmenge variiert in Abhängigkeit z. B. vom Gewicht des Patienten, vom Applikations weg, der Indikation und dem Schweregrad der Erkrankung. Unter Berücksichtigung dieser Faktoren beträgt der Wirkstoffgehalt pro Einzeldosis im allgemeinen etwa 0,01 -50 mg, und zwar bei Zubereitungsformen für die parenterale Applikation 0,01 bis 10 mg, bei Zubereitungen für orale oder rektale Applikation etwa 0,1 bis 50 mg.

Arzneimittel zur parenteralen Applikation können sowohl Lösungen als auch Suspensionen darstellen, es kommen aber auch leicht rekonstituierbare Trockenzubereitungen in Betracht.

Gut geeignete Applikationsformen sind auch Sprays zur intranasalen oder oralen Applikation bzw. zur Verabreichung der Substanzen über die Bronchien.

Für viele prophylaktische oder therapeutische Anwendungen kommen zweckmäßig auch oral anwendbare Zubereitungsformen der Verbindungen der Formel I, wie Tabletten, Dragees, Kapseln, Granulate, Tropfen und Säfte bzw. Sirupe, aber auch Suppositorien sowie perkutane Applikationszubereitungen (wie z. B. die Wirkstoffe in einem Depot in gelöster Form, gegebenenfalls unter Zusatz von die Hautpenetration fördernden Mitteln enthaltende Pflaster oder dergleichen) in Betracht. Vorteilhaft werden diese oral, rektal oder perkutan anwendbaren Zubereitungsformen so hergestellt, daß daraus der Wirkstoff verzögert freigesetzt wird, um so über einen längeren Zeitraum (beispielsweise 24 Stunden) eine gleichförmige Versorgung des Patienten mit dem Wirkstoff zu gewährleisten.

Alle vorstehend erwähnten pharmazeutischen Zubereitungsformen sind an sich bekannt, und da die erfindungsgemäßen Verbindungen der Formel I chemisch recht stabil sind, wirft ihre Einarbeitung in diese Zubereitungsformen für den Fachmann keinerlei Probleme auf. Bei dieser erfindungsgemäßen Herstellung dieser Arzneimittel muß selbstverständlich mit der üblichen Sorgfalt bei Auswahl der Hilfsstoffe wie Trägermaterialien, Farbstoffe, Geschmackskorrigentien, Bindemitteln, Tablettensprengmitteln usw. vorgegangen werden, und insbesondere bei der Herstellung von parenteral anzuwendenden Zubereitungsformen ist auf Sterilität und -wenn sie in flüssiger Form vorliegen -Isotonie zu achten.

Die erfindungsgemäße Herstellung der Verbindungen der Formel I erfolgt in der Weise, daß man

1) bei Temperaturen von etwa -80° C bis +50° C in Gegenwart eines inerten, zweckmäßig eines dipolaren aprotischen Lösungsmittels und vorzugsweise in Gegenwart katalytischer Mengen eines Kupfersalzes

a) wenn A für -C≡C-stehen soll, eine Verbindung der Formel

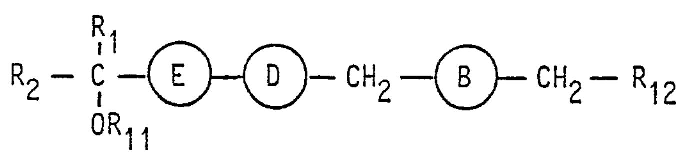

IIIa

in der B, D, E, $R_1$ und $R_2$ die gleiche Bedeutung wie in Formel I haben, $R_{11}$ eine unter milden Bedingungen abspaltbare Schutzgruppe wie einen Tetrahydropyranyl-2-rest oder insbesondere eine tert-Butyldimethyl-oder -diphenylsilygruppe darstellt und $R_{12}$ ein

Brom-oder Jodatom oder eine Toluolsulfonyloxygruppe bedeutet

mit einer Verbindung der Formel

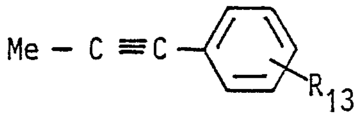

IVa

worin Me für ein Lithiumatom oder einen der Reste BrMg-oder JMg-steht und

$R_{13}$ die gleiche Bedeutung wie $R_4$ mit der Maßgabe hat, daß darin $R_7$ nicht für ein Kation stehen kann und die in $R_4$ gegebenenfalls vorhandenen basischen Gruppen nicht in Salzform vorliegen können,

umsetzt, oder

b) wenn E (und definitionsgemäß damit auch A) für $-C \equiv C-$stehen soll, eine Verbindung der Formel

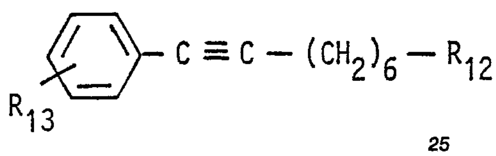

IIIb

worin $R_{12}$ und $R_{13}$ die gleiche Bedeutung wie oben haben

mit einer Verbindung der Formel

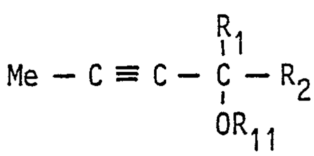

IVb

in der $R_1$, $R_2$, $R_{11}$ und Me die gleiche Bedeutung wie oben haben

umsetzt •

wobei man eine Verbindung der Formel

II

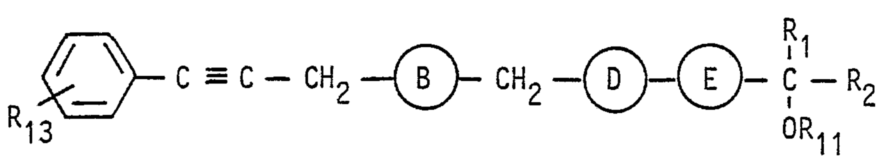

in der B, D, E, $R_1$, $R_2$, $R_{11}$ und $R_{13}$ wie oben definiert sind

erhält.

Steht in den Formeln IVa bzw. IVb der Rest Me für ein Lithiumatom, so erfolgen die betreffenden Umsetzungen vorzugsweise bei etwa -80° C bis 0° C, ist dagegen Me einer der Reste BrMg-oder IMg-,

so wird die Reaktion vorzugsweise bei etwa +5° C bis +25° C durchgeführt. Als Lösungsmittel werden inerte Lösungsmittel wie aliphatische Kohlenwasserstoffe oder vorzugsweise wasserfreie Ether wie Diethyl-oder Diiosopropylether, Tetrahydrofuran, Dioxan usw. verwendet. Insbesondere wenn Me für ein Lithiumatom steht, wird zweckmäßig zu einer Lösung der Verbindung der Formel IVabzw. IV b in einem der genannten Lösungsmittel langsam eine Lösung der Verbindung der Formel IIIa bzw. IIIb in Hexamethylphosphorsäuretriamid, 1,3-Dimethyltetrahydro-2(1H)-pyrimidinon, 1,3-Dimethyl-2-imidazolidinon oder z. B. in N,N,N',N'-Tetraethylsulfamid bzw. entsprechend dipolaren aprotischen Lösungsmitteln zugegeben.

Die Reaktion erfolgt besonders zweckmäßig in Gegenwart von katalytischen Mengen von Kupfer-(I)-halogeniden, Kupfer-(I)-cyanid oder sonstigen Kupfersalzen.

Zweckmäßig geht man bei den im Rahmen dieser Anmeldung (also vorstehend und in den folgenden Abschnitten) beschriebenen Syntheseverfahren von jeweils solchen Ausgangsmaterialien aus, in denen die gegebenenfalls vorhandenen Doppelbindungen bereits in der im Endprodukt gewünschten Konfiguration vorliegen. Dadurch wird die Isomerentrennung in den Fällen, in denen in der letzten Stufe eine weitere Doppelbindung eingeführt wird, diese aber nicht mit einheitlicher Konfiguration anfällt, erleichtert. Die Isomerentrennung kann in an sich bekannter Weise z. B. durch Säulenchromatographie erfolgen.

Aus der erhaltenen Verbindung der Formel II spaltet man dann in an sich bekannter Weise den Rest $R_{11}$ ab. Stellt $R_{11}$ eine Tetrahydropyranyl-2-Gruppe dar, so erfolgt deren Abspaltung vorzugsweise in methanolischer oder ethanolischer Lösung unter Zusatz katalytischer Mengen von Pyridinium-toluol-4-sulfonat bei etwa 50° - 60° C. Stellt dagegen $R_{11}$ eine tert-Butyldimethyl-oder -diphenylsilylgruppe dar, so wird diese Gruppe vorzugsweise durch Einwirkung von Tetra-n-butylammoniumfluorid in einem inerten Lösungsmittel wie Tetrahydrofuran, Dioxan, Diethylether, Dichlormethan etc. oder von Chlorwasserstoff, gelöst in Methanol, auf die Verbindung der Formel II bei Raumtemperatur abgespalten.

Anschließend wird dann, wenn $R_3$ für einen Acetyl-oder Propionylrest stehen soll, dieser Rest in üblicher Weise, beispielsweise durch Behandlung mit einer Lösung von Essigsäure-oder Propionsäureanhydrid in Pyridin oder durch Umsetzung mit Acetyl-oder Propionylchlorid in Gegenwart eines säurebindenden Mittels, eingeführt. Man erhält so eine Verbindung der Formel

Ia

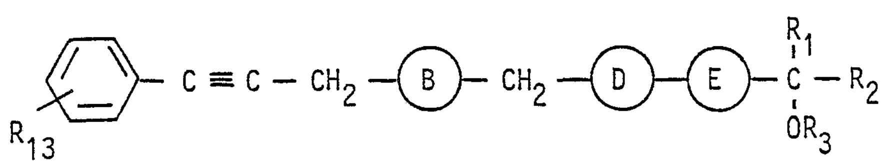

worin $R_1$ bis $R_3$, $R_{13}$, B, D und E die gleiche Bedeutung wie oben haben.

2) Eine Verbindung der Formel Ia, in der E für -C≡C-sowie B und D für $-CH_2-CH_2-$ stehen, kann auch hergestellt werden, indem man eine Verbindung der Formel

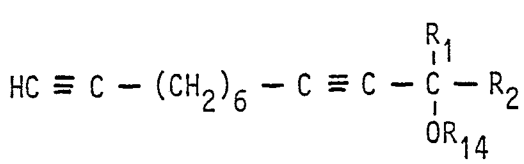

V

in der $R_1$ und $R_2$ die gleiche Bedeutung wie oben haben und $R_{14}$ für ein Wasserstoffatom steht oder die gleiche Bedeutung wie $R_{11}$ hat mit einer Verbindung der Formel

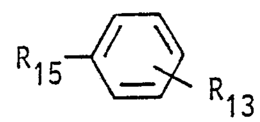

VI

worin $R_{13}$ die gleiche Bedeutung wie oben hat und $R_{15}$ für ein Brom-oder Jodatom steht

in Gegenwart eines bei etwa -10° C bis +80° C flüssigen sekundären oder tertiären Amins, insbesondere eines Dialkyl-oder Trialkylamins mit 2 oder 3 Kohlenstoffatomen in jedem der Alkylreste, Pyrrolidin oder Piperidin unter Zugabe katalytischer Mengen (darunter sollen Mengen von etwa 0,01 bis 1 Prozent pro Mol eingesetzter Verbindung der Formel V verstanden werden) eines komplexen Palladiumkatalysators, insbesondere Bis-(triphenylphosphin)-palladium-(II)-chlorid oder -acetat oder Tetrakis-(triphenylphosphin)-palladium, und gegebenenfalls unter Zusatz katalytischer Mengen Kupfer-(I)-jodid bei etwa 0° C bis 75° C umsetzt. Man erhält so eine Verbindung der Formel

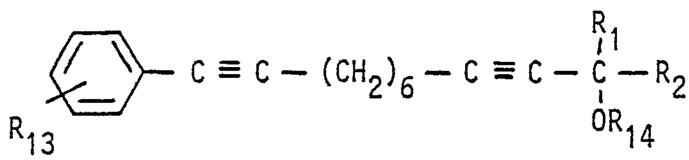

IIa

worin $R_1$, $R_2$, $R_{13}$ und $R_{14}$ die gleiche Bedeutung wie oben haben, aus der in der für die Überführung der Verbindung der Formel II in die Verbindung der Formel Ia beschriebenen Weise der Rest $R_{14}$, wenn er nicht für Wasserstoff steht, abgespalten wird und dann gegebenenfalls, wenn $R_3$ von Wasserstoff verschieden sein soll, in der oben beschriebenen Weise eine Acetyl-oder Propionylgruppe an die Hydroxylgruppe geknüpft wird, wodurch man die Verbindung der Formel Ia erhält.

Die Verbindung der Formel Ia läßt sich weiterhin dadurch erhalten, daß man

3) eine Verbindung der Formel

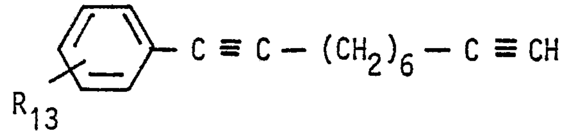

VII

worin $R_{13}$ die gleiche Bedeutung wie oben hat

in Gegenwart eines tertiären Amins, insbesondere eines Trialkylamins mit 2 oder 3 Kohlenstoffatomen in jedem der Alkylreste unter den vorstehend bei 2 für die Reaktion zwischen den Verbindungen der Formeln V und VI beschriebenen Bedingungen mit einer Verbindung der Formel

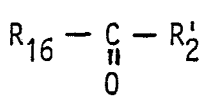

VIII

worin $R'_2$ mit Ausnahme von Wasserstoff die gleiche Bedeutung wie $R_2$ hat und $R_{16}$ für ein Chlor-, Brom-oder Jodatom steht

zu einer Verbindung der Formel umsetzt

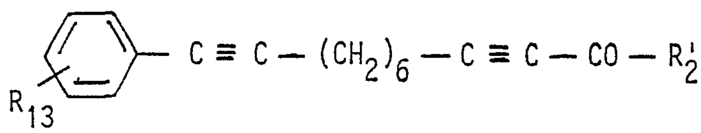

IX

worin $R'_2$ und $R_{13}$ die gleiche Bedeutung wie oben haben.

Durch Reduktion oder durch Umsetzung mit einer Verbindung der Formel $R'_1$ -Me, worin Me die gleiche Bedeutung wie oben hat und $R'_1$ mit Ausnahme von Wasserstoff die gleiche Bedeutung wie $R_1$ hat, erhält man dann aus der Verbindung der Formel IX -gegebenenfalls nach Einführung eines von Wasserstoff verschiedenen Restes $R_3$ -die entsprechende Verbindung der Formel Ia (in der $R_2$ und gegebenenfalls $R_1$ nicht für Wasserstoff stehen können).

Die Reduktion der Verbindung der Formel IX erfolgt mit Metallborhydriden, wie Zinkborhydrid oder insbesondere Natriumborhydrid, vorzugsweise unter Zusatz von Cer(III)-chlorid in Methanol, Ethanol oder auch Dimethoxyethan unter Zusatz von Wasser bei Temperaturen von etwa -20° C bis +30° C, bevorzugt bei etwa +20° C.

Zur Herstellung der entsprechenden Verbindung der Formel I wird dann in der nach 1, 2 oder 3 erhaltenen Verbindung der Formel Ia in an sich bekannter Weise der Rest $R_{13}$ in den gewünschten Rest $R_4$ übergeführt, und gegebenenfalls werden eine oder mehrere der darin vorliegenden Dreifachbindungen (außer wenn A und E für -C≡C-stehen) zu Doppelbindungen hydriert.

Diese Hydrierung erfolgt im allgemeinen mit katalytisch angeregtem Wasserstoff unter Normaldruck bei Raumtemperatur unter Verwendung insbesondere von Palladiumkatalysatoren, vorzugsweise "Lindlar-Katalysatoren" wie z. B. mit Blei vergiftetem Palladium auf Calciumcarbonat. Dabei werden als Lösungsmittel in an sich bekannter Weise z. B. n-Hexan, Methanol, Ethanol, Essigsäureethylester, Benzol, Toluol, Diisopropylether, zweckmäßig unter Zusatz von 0,1 -2 % Chinol in bzw. Pyridin oder reines Pyridin verwendet.

Die Überführung des Restes $R_{13}$ in den Rest $R_4$ erfolgt, wenn $R_{13}$ eine freie Carboxylgruppe darstellt, in üblicher Weise durch Neutralisation mit einer in Salzform pharmazeutisch verträglichen Base, insbesondere verdünnter (z. B. 1-normaler) Natron-oder Kalilauge. Zur Herstellung derartiger Salze aus Verbindungen der Formel Ia, in denen $R_{13}$ für eine in der definierten Weise veresterte Carboxylgruppe steht, muß der Neutralisation eine Verseifungsreaktion vorangehen, bei der diese Ester mit einer wässrigen Lösung von z. B. Natrium-, Kalium-oder Lithiumhydroxid, zweckmäßig bei Raumtemperatur in Gegenwart eines mit Wasser mischbaren Lösungsmittels wie Methanol, Ethanol oder Tetrahydrofuran behandelt werden.

Aus den erhaltenen Estern der Formel Ia lassen sich durch Umsetzung mit Aminen der Formeln

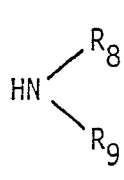 , $H_2N-(CH_2)_r-N(CH_3)_2$ oder 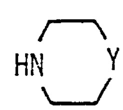

worin $R_8$, $R_9$, r und Y die gleiche Bedeutung wie oben haben,

bzw. mit Hydroxylaminabkömmlingen der Formel

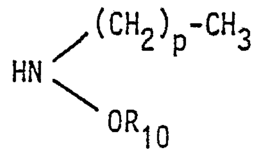

worin p und $R_{10}$ die gleiche Bedeutung wie oben haben,

die entsprechenden Amide bzw. Hydroxamsäuren gewinnen, die andererseits natürlich in an sich bekannter Weise auch aus den freien Carbonsäuren - ($R_{13}$ bzw. $R_4$ = COOH) unter Zuhilfenahme wasserabspaltender Mittel wie Dicyclohexylcarbodiimid, Dimethylformamid/Thionylchlorid und dergleichen oder durch intermediäre Bildung reak tiver funktioneller Derivate des Carbonsäurerestes, wie z. B. gemischter Anhydride, Säurehalogenide usw., zugänglich sind.

Enthält in den auf einem der obigen Wege gewonnenen Verbindungen der Formel I bzw. Ia der Rest $R_4$ bzw. $R_{13}$ eine basische Gruppe, so läßt sich diese leicht durch Neutralisation mit einer geeigneten Säure in an sich bekannter Weise in ein pharmazeutisch verträgliches Salz mit dieser Säure überführen. Erfolgte diese Salzbildung in einem Lösungsmittel, in dem das entstehende Salz löslich ist, so erfolgt dessen Isolierung zweckmäßig durch Gefriertrocknung, obwohl natürlich auch das Ausfällen durch Zugabe von geeigneten, mit dem Lösungsmittel mischbaren Flüssigkeiten, in denen das Salz unlöslich ist, zur Isolierung des Salzes dienen kann. Vorzugsweise löst man aber die freie Base in einem wasserfreien, wenig polaren Mittel, wie z. B. Diethylether, gibt eine etherische Lösung der Säure zu und versetzt gegebenenfalls zur Einleitung der Kristallisation beispielsweise mit n-Hexan oder Petrolether. Auch in diesem Falle ist andererseits selbstverständlich die Isolierung des Salzes durch Eindampfen, vorzugsweise im Vacuum, möglich. Geeignete Säuren für diese Salzbildung sind z. B. Salzsäure, Bromwasserstoffsäure, Schwefelsäure, Phosphorsäure, Ameisensäure, Essigsäure, Benzoesäure, Salicylsäure, Benzolsulfonsäure und weitere zur Herstellung pharmazeutisch anwendbarer Salze gebräuchliche Säuren.

Weitere Umwandlungsmöglichkeiten des Restes $R_4$ bzw. $R_{13}$ liegen für den Fachmann auf der Hand. Beispielsweise kann die freie Carboxylgruppe (insbesondere wenn $R_3$ von Wasserstoff verschieden ist) leicht durch Behandlung mit Diazomethan oder Diazoethan in die Carboxymethyl-bzw. Carboxyethylgruppe übergeführt werden.

Die Darstellung der Ausgangsverbindungen für die oben beschriebenen Vorgehensweisen zur Herstellung der Verbindungen der Formeln II bzw. Ia erfolgt durchweg in an sich bekannter Weise. Die dafür in Betracht zu ziehenden Wege werden insbesondere auch unter Berücksichtigung der verschiedenen Bedeutungen von B, D und E gewählt. Z. B. kann man zur Herstellung einer Verbindung der Formel IIIa, in der E für -C≡C-sowie B und D für $-CH_2-CH_2-$stehen, eine Verbindung der Formel IVb mit 1,6-Dibromhexan oder 1,6-Dijodhexan umsetzen, wobei dann eine Verbindung der Formel IIIa, in der $R_{12}$ für Brom oder Jod steht, anfällt. Diese Umsetzung erfolgt in der oben für die Reaktion der Verbindung der Formel IVb mit der Verbindung der Formel IIIb beschriebenen Weise.

Eine Verbindung der Formel IIIb kann in entsprechender Weise leicht durch Reaktion einer Verbindung der Formel IVa mit einer Verbindung der Formel

$R_{12}$ $-(CH_2)_6$ $-R_{12}$ X

z. B. unter Verwendung von Diethylether oder Tetrahydrofuran als Lösungsmittel, vorzugsweise in Anwesenheit eines aprotischen dipolaren Lösungsmittels bei etwa -80° C, später bei 0° C, erhalten werden.

Verbindungen der Formel V lassen sich leicht aus der wie oben beschrieben hergestellten Verbindung der Formel IIIa, in der E für -C≡C-, B und D für $-CH_2-CH_2-$sowie $R_{12}$ für Brom oder Jod stehen, durch Umsetzung mit Lithiumacetylid-Ethylendiamin-Komplex in Dimethylsulfoxid bei Temperaturen von etwa 10° C und gegebenenfalls nachfolgende Abspaltung der Schutzgruppe $R_{11}$ erhalten.

Die Herstellung einer Verbindung der Formel VII gelingt leicht durch Umsetzung von Deca-1,9-diin mit einer Verbindung der Formel VI in Gegenwart eines sekundären oder tertiären Amins, bevorzugt Triethylamin, in Gegenwart katalytischer Mengen von Kupfer-(I)-jodid und eines komplexen Palladium-katalysators, wie z. B. Bis-(triphenylphosphin)-palladium-(II)-chlorid bei etwa 0° C bis 60° C, bevorzugt 20° C bis 25° C.

Weitere Herstellungsmöglichkeiten für die Ausgangsverbindungen für das erfindungsgemäße Verfahren zur Herstellung von Verbindungen der Formel I ergeben sich für den Fachmann anhand der vorstehenden Ausführungen und des Standes der Technik in naheliegender Weise. Darüberhinaus sind verschiedene der erwähnten Ausgangsmaterialien auch literaturbekannt und zum Teil auch im Handel zugänglich.

Die folgenden Beispiele dienen zur näheren Erläuterung des erfindungsgemäßen Verfahrens sowie der Vorgehensweise zur Herstellung der dabei benutzten neuen Ausgangsmaterialien und Zwischenprodukte. Bei ihrer Durchführung wurde auf die Erzielung maximaler Ausbeuten kein Wert gelegt.

Alle Temperaturangaben sind unkorrigiert.

Die Produkte liegen in Form von Ölen vor, soweit im Einzelfall keine anderen Angaben gemacht werden.

Die $^1$H-NMR-Spektren wurden bei 60 MHz mit dem Gerät WP-60 der Firma Bruker aufgenommen. Die chemische Verschiebung der spektroskopischen Resonanzdaten wurde in ppm gemessen.

Die in den Beispielen verwendete n-Butyllithiumlösung enthielt -gelöst in n-Hexan -1,6 Mol n-Butyllithium/Liter. Es können aber selbstverständlich auch Lösungen anderer Konzentrationen und/oder mit anderen Lösungsmitteln als n-Hexan eingesetzt werden.

Die dünnschichtchromtographische Verfolgung des Reaktionsverlaufs wurde mit "HPTLC Fertigplatten, Kieselgel 60 F 254" der Firma E. Merck, Darmstadt, durchgeführt. Das benutzte Fließmittel wird in den Beispielen jeweils mit "(DC: ...)" angegeben.

Für die Chromatographie bzw. Säulenchromatographie wurde als stationäre Phase -sofern nicht anders erwähnt -Kieselgel 60 (0,040 - 0,063 mm) der Firma Macherey-Nagel benutzt.

Für die HPLC wurde ein von Macherey-Nagel unter der Bezeichnung "Nucleosil C 18 (10 µm)" vertriebenes Kieselgel als stationäre Phase eingesetzt.

Soweit nicht anders angegeben, handelt es sich bei dem in den Beispielen benutzten Ether um den Diethylether, bei dem Petrolether um denjenigen mit einem Siedebereich von 50° -70° C.

Die Mischungsverhältnisse der Laufmittel für alle chromatographischen Untersuchungen sind stets in Volumen/Volumen angegeben.

Soweit nicht anders erwähnt, wurden in den Beispielen anfallende Enantiomerengemische nicht aufgetrennt. Die angegebenen Daten beziehen sich in solchen Fällen also auf die Enantiomerengemische.

Referenzbeispiel A

(Reaktion eines 2-Oxoalkyl-phosphonsäureesters nach Wittig-Horner mit einem Aldehyd am Beispiel der Herstellung von 5-Oxo-3E-decen-1-in).

Eine Lösung von 26,7 g (2-Oxoheptyl)-phosphonsäuredimethylester in 500 ml absolutem Tetrahydrofuran wird unter Rühren und Überleiten von trockenem Stickstoff tropfenweise so mit 68,8 ml n-Butyllithiumlösung versetzt, daß dabei die Innentemperatur 5° C nicht übersteigt. Man rührt 15 Minuten nach und tropft dann bei derselben Temperatur 5,4 g Propiolaldehyd in 250 ml absolutem Dimethoxyethan zu. Danach rührt man das Reaktionsgemisch noch 1 Stunde weiter und läßt dabei auf Raumtemperatur erwärmen. Anschließend wird durch tropfenweise Zugabe von Essigsäure neutralisiert und im Vakuum eingedampft. Man nimmt den Rückstand in Ether auf, wäscht die Lösung je zweimal mit gesättigten Lösungen von Natriumhydrogencarbonat bzw. Natriumchlorid und trocknet über Natriumsulfat.

Das nach Eindampfen im Vakuum erhaltene Rohprodukt wird durch Säulenchromatographie mit Petrolether/Ether (5 : 1) gereinigt. Man erhält 10,82 g der Titelverbindung.

Referenzbeispiel B

(Reduktion einer Carbonylgruppe in Ketonen mit zusätzlicher Doppel-und gegebenenfalls Dreifachbindung am Beispiel der Herstellung von 5-Hydroxy-3E-decen-1-in)

Zu 10,51 g 5-Oxo-3E-decen-1-in in 175 ml einer Lösung von Cer(III)-chlorid in Methanol (0,4 Mol/l) gibt man bei Raumtemperatur unter Rühren und Überleiten von trockenem Stickstoff vorsichtig und in kleinen Portionen 2,65 g Natriumborhydrid. Es wird etwa 30 Minuten nachgerührt, dann durch Zufließenlassen von 70 ml Pufferlösung-pH 7 zersetzt. Man versetzt mit 250 ml Dichlormethan und 50 ml einer gesättigten Kaliumnatriumtartratlösung. Die organische Phase wird abgetrennt, die wässrige noch zweimal mit je 50 ml Dichlormethan extrahiert. Die vereinigten organischen Phasen wäscht man je zweimal mit gesättigten Lösungen von Natriumhydrogencarbonat bzw. Natriumchlorid und trocknet über Natriumsulfat. Das durch Eindampfen im Vakuum erhaltene Rohprodukt wird durch Säulenchromatographie mit Petrolether/Ether (2 : 1) gereinigt. Man erhält 9,81 g der Titelverbindung.

Beispiel 1

3-(11'-Hydroxy-hexadec-9'E-en-1',4',7'-triin-1'-yl)-benzoesäuremethylester.

a) 5-(tert-Butyldiphenylsilyloxy)-3E-decen-1-in

18,2 ml tert-Butyldiphenylchlorsilan werden bei 0° C unter Rühren und Überleiten von trockenem Stickstoff tropfenweise zu einer Lösung von 8,83 g 5-Hydroxy-3E-decen-1-in (vgl. Referenzbeispiel B) und 4,77 g Imidazol in 60 ml absolutem Dimethylformamid gegeben. Nach beendeter Zugabe wird 3 Stunden bei Raumtemperatur weitergerührt, dann mit Dichlormethan verdünnt und nacheinander mit Wasser sowie mit gesättigten Lösungen von Natriumhydrogencarbonat bzw. Natriumchlorid gewaschen. Die organische Phase wird über Natriumsulfat getrocknet. Nach Eindampfen im Vakuum wird das zurückbleibende Öl durch Säulenchromatographie mit Petrolether/Toluol (10 : 1) gereinight. Man erhält 19,48 g der Titelverbindung.

$^1$H-NMR ($CDCl_3$):

0,63 -1,65 (m, 11 H); 1,10 (s, 9 H); 2,80 (d, 1 H); 3,95 -4,36 (m, 1 H); 5,23 -6,33 (m, 2 H); 7,05 -7,75 (m, 10 H.

b) 1-(p-Toluolsulfonyloxy)-9-(tert-butyldiphenylsilyloxy)-7E-tetradecen-2,5-diin.

Aus 0,316 g Magnesiumspänen und 0,93 ml Ethylbromid in 10 ml absolutem Tetrahydrofuran wird unter Überleiten von trockenem Stickstoff eine Grignard-Lösung bereitet. Dazu wird unter Rühren bei Raumtemperatur eine Lösung von 3,91 g 5-(tert-Butyl-diphenylsilyloxy)-3E-decen-1-in in 10 ml absolutem Tetrahydrofuran so zugetropft, daß die Innentemperatur 25° C nicht übersteigt. Man rührt noch 15 Minuten weiter, nimmt dann die gelb gefärbte Lösung mit einer Spritze auf und tropft sie unter Rühren und Überleiten von trockenem Stickstoff so zu einer Lösung von 5,92 g 1,4-Bis-(p-toluolsulfonyloxy)-2-butin (G. Eglington et al., J. Chem. Soc. 1950, 3650) in 15 ml absolutem Tetrahydrofuran, die außerdem ca. 50 mg Kupfer(I)-cyanid enthält, daß die Temperatur 30° C nicht übersteigt. Man rührt noch 30 Minuten nach, gießt dann in ein Gemisch aus 20 ml gesättigter Ammoniumchloridlösung und 40 ml Ether, trennt die organische Phase ab und extrahiert die wässrige Schicht noch zweimal mit Ether. Die vereinigten organischen Phasen wäscht man dreimal mit gesättigter Natriumchloridlösung und trocknet über Natriumsulfat. Eindampfen im Vakuum liefert 7,64 g eines Öls, das durch Säulenchromatographie mit Petrolether/ Ether (7 : 3) gereinigt wird. Man erhält 2,86 g der Titelverbindung.

$^1$H-NMR ($CDCl_3$):

0,60 -1,60 (m, 11 H); 1,10 (s, 9 H); 2,43 (s, 3 H); 3,05 -3,25 (m, 2 H); 3,96 -4,30 (m, 1 H); 4,56 - 4,73 (t, 2 H); 5,16 -6,20 (m, 2 H); 7,03 -7,83 (m, 14 H).

c) 3-[11'-(tert-Butyl-diphenylsilyloxy)-hexadec-9'E-en-1',4',7'-triin-1'-yl]-benzoesäuremethylester

I 1,40 g 3-Ethinyl-benzoesäuremethylester [J. Org. Chem. 31, 2585 (1966)] werden in 10 ml absolutem Tetrahydrofuran gelöst. Zu der Lösung gibt man unter trocknem Stickstoff vorsichtig bei 0 -2° C 12,79 ml einer frisch bereiteten Lösung von Ethylmagnesiumbromid (0,833 Mol/l) in Tetrahydrofuran. Nach erfolgter Grignardzugabe wird noch 90 Minuten im Eisbad nachgerührt. Das Reaktionsgemisch wird mit 0,20 g Kupfer(I)-bromid und anschließend innerhalb von 30 Minuten mit 2,68 g des in Beispiel 1b erhaltenen Produktes in 16 ml absolutem Tetrahydrofuran versetzt. (DC: n-Hexan/Essigsäureethylester/Ether -3 : 0,1 : 0,1). Nach etwa 2 Stunden wird mit gesättigter Ammoniumchloridlösung versetzt und dann mit Essigsäureethylester extrahiert. Die organische Phase wird mit gesättigter Natriumchloridlösung gewaschen, über Natriumsulfat getrocknet und dann im Vakuum eingedampft, wobei ein Öl hinterbleibt. Durch Säulenchromatographie mit n-Hexan/Essigsäureethylester/Ether (3 : 0,1 : 0,1) erhält man daraus 1,51 g der Titelverbindung.

$^1$H-NMR ($CDCl_3$):

0,60 -1,53 (m, 11 H); 1,10 (s, 9 H); 3,16 -3,47 (m, 4 H); 3,90 (s, 3 H); 3,93 -4,30 (m, 1 H); 5,20 - 6,23 - (m, 2 H); 7,07 -8,07 (m, 14 H).

Das gleiche Produkt kann auch folgendermaßen erhalten werden:

II i) 1-Hydroxy-6-(tert-butyldiphenylsilyloxy)-4E-undecen-2-in

6,84 g 5-(tert-Butyldiphenylsilyloxy)-3E-decen-1-in werden in 50 ml absolutem Ether gelöst und tropfenweise bei -40° C unter Rühren und Überleiten von trockenem Stickstoff mit 10,9 ml n-Butyllithiumlösung versetzt. Es wird noch 30 Minuten weitergerührt, wobei man auf Raumtemperatur erwärmen läßt. Dann gibt man 1,05 g Paraformaldehyd zu und erhitzt 3 Stunden zum Rückfluß. Man läßt abkühlen, gießt auf 20 ml gesättigte Ammoniumchloridlösung, verdünnt mit 50 ml Ether und trennt die organische Phase ab. Die wässrige Phase wird nochmals ausgeethert. Die vereinigten organischen Phasen werden zweimal mit gesättigter Natriumchloridlösung gewaschen, über

Natriumsulfat getrocknet und im Vakuum eingedampft. Der Rückstand wird durch Säulenchromatographie mit Petrolether/Ether (3 : 2) gereinigt. Man erhält 5,74 g der Titelverbindung.

$^1$H-NMR ($CDCl_3$):

0,53 -1,60 (m, 11 H); 1,05 (s, 9 H); 1,53 (s, 1 H); 3,95 -4,36 (m 1 H); 4,30 (d, 2 H); 5,35 -6,20 (m, 2 H); 7,13 -7,75 (m, 10 H).

ii) 1-(p-Toluolsulfonyloxy)-6-(tert-butyl-diphenylsilyloxy)-4E-undecen-2-in

5,68 g des Produktes aus i) und 2,96 g p-Toluolsulfonsäurechlorid werden in 20 ml absolutem Ether gelöst und bei 0° C unter Rühren und Überleiten von trockenem Stickstoff portionsweise mit 0,99 g pulverisiertem Kaliumhydroxid versetzt. Dann läßt man in etwa 30 Minuten auf Raumtemperatur erwärmen und gibt dann portionsweise weitere 0,53 g Kaliumhydroxid hinzu. Es wird noch 2 Stunden gerührt, danach filtriert, mit absolutem Ether gewaschen und das Filtrat im Vakuum eingedampft. Dabei erhält man 7,10 g der Titelverbindung in für die Weiterverarbeitung genügend reiner Form.

$^1$H-NMR ($CDCl_3$):

0,65 -1,55 (m, 11 H); 1,07 (s, 9 H); 2,37 (s, 3 H); 3,95 -4,30 (m, 1 H); 4,76 (d, 2 H); 5,13 -6,15 (m, 2 H); 7,06 -7,86 (m, 14 H).

iii) 1-Jod-6-(tert-butyldiphenylsilyloxy)-4E-undecen-2-in

7,01 g des vorstehend in ii) erhaltenen Produktes, gelöst in 25 ml absolutem Aceton, werden unter Rühren und Überleiten von trockenem Stickstoff zu einer Lösung von 4,57 g Natriumjodid in 25 ml absolutem Aceton getropft. Nach Ablauf der Reaktion unter Rühren bei Raumtemperatur wird filtriert, mit Aceton nachgewaschen und das Filtrat im Vakuum eingedampft. Den Rückstand nimmt man in 100 ml Ether/30 ml Wasser auf und trennt die Etherphase ab. Diese wird noch zweimal mit Wasser gewaschen, über Natriumsulfat getrocknet und dann im Vakuum eingedampft. Das zurückbleibende Öl wird durch Chromatographie über Kieselgel 60 (0,063 -0,200 mm) mit Petrolether/Ether (10 : 1) gereinigt. Man erhält 5,89 g der Titelverbindung.

$^1$H-NMR ($CDCl_3$):

0,65 - 1,60 (m, 11 H); 1,06 (s, 9 H); 3,80 (d, 2 H); 3,95 -4,30 (m, 1 H); 5,23 -6,25 (m, 2 H); 7,13 -7,70 (m, 10 H).

iv) 1-(Tetrahydro-2'-pyranyloxy)-9-(tert-butyl-diphenylsilyloxy)-7E-tetradecen-2,5-diin

Zu einer Grignard-Lösung aus 0,243 g Magnesiumspänen und 0,71 ml Ethylbromid in 8 ml absolutem Tetrahydrofuran wird bei Raumtemperatur unter Rühren und Überleiten von trocknem Stickstoff langsam eine Lösung von 1,12 g 3-(Tetrahydro-2'-pyranyloxy)-1-propin (H. B. Henbest et al., J. Chem. Soc. 1950, 3646) in 8 ml absolutem Tetrahydrofuran getropft. Man rührt etwa 45 Minuten nach und fügt dann ca. 50 mg Kupfer-(I)-cyanid hinzu. Nach weiteren 15 Minuten gibt man 3,45 g 1-Jod-6-(tert-butyl-diphenylsilyloxy-4E-undecen-2-in, gelöst in 6,5 ml absolutem Tetrahydrofuran, tropfenweise zu. (DC: n-Hexan/Essigsäureethylester -6 : 1). Nach etwa 2 Stunden wird mit 10 ml gesättigter Ammoniumchloridlösung zersetzt und das Gemisch mehrfach mit Ether extrahiert. Die organische Phase wird mit gesättigter Natriumchloridlösung gewaschen und dann über Natriumsulfat getrocknet. Eindampfen im Vakuum liefert 2,80 g eines Öls, aus dem man durch Säulenchromatographie mit n-Hexan/Essigsäureethylester (6 : 1) 1,81 g der reinen öligen Titelverbindung erhält.

$^1$H-NMR ($CDCl_3$):

0,60 -1,95 (m, 17 H); 1,06 (s, 9 H); 3,16 -3,40 (m, 2 H); 3,40 -4,25 (m, 3 H); 4,10 -4,30 (m, 2 H); 4,60 - 4,83 (m, 1 H); 5,16 -6,20 (m, 2 H); 7,10 -7,73 (m, 10 H).

v) 1-Hydroxy-9-(tert-butyldiphenylsilyloxy)-7E-tetradecen-2,5-diin

Eine Lösung von 1,74 g des Produktes aus iv) und 0,08 g Pyridinium-toluol-4-sulfonat in 25 ml absolutem Ethanol wird unter Überleiten von trockenem Stickstoff 2 Stunden lang bei 55° C Badtemperatur gerührt. Dann wird im Vakuum eingedampft und der Rückstand mit Petrolether/Ether (2 : 1) chromatographiert. Man erhält 1, 25 g der Titelverbindung.

$^1$H-NMR ($CDCl_3$):

0,60 -1,70 (m, 12 H); 1,06 (s, 9 H); 3,16 -3,40 (m, 2 H); 4,05 -4,36 (m, 3 H); 5,23 -6,23 (m, 2 H); 7,15 - 7,75 (m, 10 H).

vi) 1-Brom-9-(tert-butyldiphenylsilyloxy)-7E-tetradecen-2,5-diin

1,24 g 1-Hydroxy-9-(tert-butyldiphenylsilyloxy)-7E-tetradecen-2,5-diin und 1,42 g Triphenylphosphin werden in 15 ml absolutem Dichlormethan gelöst. Dazu gibt man bei 0°C unter Rühren und Überleiten von trockenem Stickstoff portionsweise 1,79 g Tetrabrommethan. Nach einstündigem Rühren wird über Kieselgel 60 (0,063 -0,200 mm) mit n-Hexan chromatographiert und nach Eindampfen der Lösung im Vakuum der Rückstand durch Säulenchromatograhie mit n-Hexan/Ether (20 : 1) gereinigt. Man erhält 1,20 g der öligen Titelverbindung.

$^1$H-NMR ($CDCl_3$):

0,60 -1,60 (m, 11 H); 1,06 (s, 9 H); 3,23 -3,43 (m, 2 H); 3,80 -3,96 (t, 2 H); 3,93 -4,33 (m, 1 H); 5,23 - 6,25 (m, 2 H); 7,15 -7,75 (m, 10 H).

vii) Man verfährt analog Beispiel 1cI und erhält aus 2,90 g 3-Ethinylbenzoesäuremethylester, 3,80 g 1-Brom-9-(tert-butyldiphenylsilyloxy)-7E-tetradecen-2,5-diin und 0,25 g Kupfer(I)-cyanid 2,28 g eines Öls, dessen spektroskopische Resonanzdaten mit denen des nach Beispiel 1cI hergestellten Produktes identisch sind.

An Stelle der Bromverbindung kann man bei diesem Vorgehen auch die entsprechende Jodverbindung einsetzen, die aus der Bromverbindung analog Beispiel 1cIliii durch Umsetzung mit Natriumjodid in absolutem Aceton erhältlich ist. Aus 0,93 g 3-Ethinyl-benzoesäuremethylester, 1,32 g 1-Jod-9-(tert-butyldiphenylsilyloxy)-7E-tetradecen-2,5-diin und 0,17 g Kupfer-(I)-cyanid erhält man so 0,59 g eines Öls, dessen spektroskopische Resonanzdaten mit denen des nach Beispiel 1cI hergestellten Produktes identisch sind.

d) 3-(11'-Hydroxy-hexadec-9'E-en-1',4',7'-triin-1'-yl)-benzoesäuremethylester

1,34 g des in Beispiel 1c erhaltenen Produktes werden unter trockenem Stickstoff mit 15,5 ml einer methanolischen Lösung von Chlorwasserstoff (2 %) versetzt und bei Raumtemperatur gerührt bis alles in Lösung gegangen ist. (DC: n-Hexan/Ether - 1 : 1). Dann wird mit Essigsäureethylester verdünnt und nacheinander mit gesättigten Lösungen von Natriumbicarbonat und Natriumchlorid gewaschen. Es wird über Natriumsulfat getrocknet, eingedampft und der ölige Rückstand durch Säulenchromatographie mit n-Hexan/Ether (1 : 1) gereinigt, wobei man 0,59 g der Titelverbindung erhält.

$^1$H-NMR ($CDCl_3$):

0,67 -1,80 (m, 11 H); 3,07 -3,50 (m, 4 H); 3,90 (s, 3 H); 3,97 -4,27 (m, 1 H); 5,37 -6,27 (m, 2 H); 7,07 - 8,07 (m, 4 H).

Beispiel 2

3-(11'-Hydroxy-hexadeca-1'Z,4'Z,7'Z,9'E-tetraen-1'-yl)-benzoesäuremethylester

0,29 g des Beispiel 1 erhaltenen Produktes werden in 8 ml absolutem Ethanol gelöst, mit 0,1 ml frisch destilliertem Chinolin versetzt und über 0,095 g Palladium auf Calciumcarbonat (5 % Pd; mit Blei vergifteter "Lindlar-Katalysator") hydriert. Nach langsamer Aufnahme von 55 ml Wasserstoff unter Normaldruck und bei Raumtemperatur kommt die Hydrierung zum Stillstand. Man filtriert den Katalysator ab und engt das Filtrat im Vakuum ein. Der Rückstand wird durch Säulenchromatographie an Kieselgel 60 (0,063 -0,200 mm) mit n-Hexan/Ether (1 : 1) vorgereinigt. Dabei fallen 0,22 g eines Produktes an, welches durch HPLC mit Methanol/Wasser (85 : 15) weiter aufgetrennt wird. Man erhält 0,14 g der Titelverbindung.

$^1$H-NMR ($CDCl_3$):

0,57 -1,63 (m, 11 H); 1,60 (s, 1 H); 2,60 -3,10 (m, 4 H); 3,80 (s, 3 H); 3,80 -4,17 (m, 1 H); 5,00 -6,53 (m, 8 H); 7,15 -7,85 (m, 4 H).

Beispiel 3

3-(11'-Hydroxy-hexadeca-1'Z,4'Z,7'Z,9'E-tetraen-1'-yl)-benzoesäure

0,10 g des in Beispiel 2 erhaltenen Produktes, gelöst in 1 ml Methanol und 1 ml Tetrahydrofuran werden mit 1,08 ml einer wässrigen Lösung von Lithiumhydroxid (1 Mol/l) versetzt. (DC: Ether). Nach 18 Stunden wird im Vakuum weitgehend eingedampft, der Rückstand mit 2 ml Wasser verdünnt, unter Eiswasserkühlung mit verdünnter Salzsäure auf pH 3 -4 angesäuert und dreimal mit Ether extrahiert. Die vereinigten Etherextrakte werden zweimal mit Wasser gewaschen, über Natriumsulfat getrocknet und dann eingedampft. Man erhält 0,086 g der Titelverbindung.

$^1$H-NMR ($CDCl_3$):

0,43 -1,73 (m, 11 H); 2,12 -3,13 (m, 4 H); 3,83 -4,37 (m, 1 H); 4,90 -6,17 (m, 8 H); 6,77 -8,10 (m, 4 H).

Beispiel 4

3-(11'-Cyclohexyl-11'-hydroxy-undec-9'E-en-1',4',7'-triin-1'-yl)-benzoesäuremethylester

a) 5-Cyclohexyl-5-oxo-3E-penten-1-in

Man arbeitet analog Referenzbeispiel A und erhält so aus 15,88 g (2-Cyclohexyl-2-oxoethyl)-phosphonsäuredimethylester, 38,8 ml n-Butyllithiumlösung und 3,06 g Propiolaldehyd (75 %ige Lösung in Toluol) 7,78 g der Titelverbindung.

$^1$H-NMR ($CDCl_3$):

1,07 -2,10 (m, 10 H); 2,20 -2,77 (m, 1 H); 3,31 (d, 1 H); 6,53 -6,77 (m, 2 H).

b) 5-Cyclohexyl-5-hydroxy-3E-penten-1-in

Man verfährt analog Referenzbeispiel B und erhält aus 7,62 g 5-Cyclohexyl-5-oxo-3E-penten-1-in, 117,5 ml einer Lösung von Cer(III)-chlorid in Methanol (0,4 Mol/l) und 1,78 g Natriumborhydrid 7,06 g der Titelverbindung.

$^1$H-NMR ($CDCl_3$):

0,65 -2,03 (m, 11 H); 1,60 (s, 1 H); 2,87 (d, 1 H); 3,77 -4,05 (m, 1 H); 5,43 -6,43 (m, 2 H).

c) 5-Cyclohexyl-5-(tert-butyldiphenylsilyloxy)-3E-penten-1-in

Durch Anwendung der in Beispiel 1a beschriebenen Verfahrensweise erhält man aus 6,95 g 5-Cyclohexyl-5-hydroxy-3E-penten-1-in, 13,2 ml tert-Butyldiphenylchlorsilan und 3,46 g Imidazol nach Säulenchromatographie mit Petrolether/Ether (7 : 3) 13,80 g der Titelverbindung.

$^1$H-NMR ($CDCl_3$):

0,65 -1,90 (m, 11 H); 1,10 (s, 9 H); 2,75 (d, 1 H); 3,77 -4,07 (m, 1 H); 5,07 -6,30 (m, 2 H); 7,13 -7,70 (m, 10 H).

d) 1-(p-Toluolsulfonyloxy)-9-cyclohexyl-9-(tert-butyldiphenylsilyloxy)-7E-nonen-2,5-diin

Man arbeitet analog Beispiel 1b und erhält so aus 4,84 g des Produktes des Beispiels 4c, 12 ml einer frisch bereiteten Lösung von Ethylmagnesiumbromid in Tetrahydrofuran (1,25 Mol/l) und 7,10 g 1,4-Bis-(p-toluolsulfonyloxy)-2-butin 3,23 g der Titelverbindung.

$^1$H-NMR ($CDCl_3$:

0,67 -1,90 (m, 11 H); 1,06 (s, 9 H); 2,40 (s, 3 H); 3,00 -3,20 (m, 2 H); 3,77 -4,03 (m, 1 H); 4,67 (t, 2 H); 5,03 -6,15 (m, 2 H); 7,10 -7,83 (m, 14 H).

e) 3-[11'-Cyclohexyl-11'-(tert-butyldiphenylsilyloxy)-undec-9'E-en-1',4',7'-triin-1'-yl]-benzoesäuremethylester

Man verfährt analog Beispiel 1cl und erhält aus 1,60 g 3-Ethinyl-benzoesäuremethylester, 12 ml - (0,83 Mol/l) einer frisch bereiteten Lösung von Ethylmagnesiumbromid in Tetrahydrofuran, 0,23 g Kupfer-(I)-bromid und 3,13 g des Produktes aus Beispiel 4d 1,59 g der Titelverbindung.

$^1$H-NMR ($CDCl_3$):

0,67 -1,83 (m, 20 H); 3,13 -3,43 (m, 4 H); 3,73 -4,00 (m, 1 H); 3,85 (s, 3 H); 5,05 -6,15 (m, 2 H); 7,05 - 8,03 (m, 14 H).

f) 3-(11'-Cyclohexyl-11'-hydroxy-undec-9'E-en-1',4',7'-triin-1'-yl)-benzoesäuremethylester

1,23 g des in Beispiel 4e erhaltenen Produktes werden analog Beispiel 1d mit 14 ml einer methanolischen Lösung von Chlorwasserstoff (5 %) umgesetzt. Man isoliert 0,48 g der Titelverbindung.

$^1$H-NMR ($CDCl_3$):

0,75 -1,97 (m, 11 H); 1,53 (s, 1 H); 3,13 -3,43 (m, 4 H); 3,63 -3,97 (m, 1 H); 3,87 (s, 3 H); 5,37 -6,28 (m, 2 H); 7,07 -8,03 (m, 4 H).

Beispiel 5

3-(11'-Cyclohexyl-11'-hydroxy-undeca-1'Z,4'Z,7'Z,9'E-tetra-en-1'-yl)-benzoesäuremethylester

0,337 g des in Beispiel 4 erhaltenen Produktes werden analog Beispiel 2 in alkoholischer, chinolinhaltiger Lösung über 0,084 g Palladium auf Calciumcarbonat (5 % Pd, mit Blei vergiftet) hydriert. Nach Reinigung durch HPLC mit Methanol/Wasser (85 : 15) erhält man 0,182 g der Titelverbindung.

$^1$H-NMR ($CDCl_3$):

0,75 -2,10 (m, 12 H); 2,67 -3,23 (m, 4 H); 3,67 -3,97 (m, 1 H); > 3,87 (s, 3 H); 5,03 -6,57 (m, 8 H); 7,20 - 7,90 (m, 4 H).

Beispiel 6

4-(11'-Hydroxy-hexadec-9'E-en-1',4',7'-triin-1'-yl)-benzoesäuremethylester

a) 4-[11'-(tert-Butyl-diphenylsilyloxy)-hexadec-9'E-en-1',4',7'-triin-1'-yl)-benzoesäuremethylester

Man verfährt analog Beispiel 1cl und erhält aus 1,28 g 4-Ethinyl-benzoesäuremethylester [J. Org. Chem. 46, 2280 (1981)], 9,6 ml einer frisch bereiteten Lösung von Ethylmagnesiumbromid in Tetrahydrofuran (0,83 Mol/l), 0,18 g Kupfer-(I)-bromid und 2,45 g 1-(p-Toluolsulfonyloxy)-9-(tert-butyldiphenylsilyloxy)-7E-tetradecen-2,5-diin (Produkt des Beispiels 1b) 1,35 g der Titelverbindung.

$^1$H-NMR ($CDCl_3$):

0,60 -1,60 (m, 11 H); 1,07 (s, 9 H); 3,17 -3,47 (m, 4 H); 3,87 (s, 3 H); 3,90 -4,30 (m, 1 H); 5,20 -6,20 (m, 2 H); 7,07 -7,97 (m, 14 H).

b) 4-(11'-Hydroxy-hexadec-9'E-en-1',4',7'-triin-1'-yl)-benzoesäuremethylester

Man arbeitet analog Beispiel 1d und erhält aus 1,20 g des in Beispiel 6a dargestellten Produktes und 14 ml einer methanolischen Lösung von Chlorwasserstoff (2 %) 0,49 g der Titelverbindung.

$^1$H-NMR ($CDCl_3$):

0,75 -1,75 (m, 12 H); 3,23 -3,53 (m, 4 H); 3,93 (s, 3 H); 3,95 -4,33 (m, 1 H); 5,47 -6,33 (m, 2 H); 7,33 - 8,10 (m, 4 H).

Beispiel 7

4-(11'-Hydroxy-hexadeca-1'Z,4'Z,7'Z,9'E-tetraen-1'-yl)-benzoesäuremethylester

0,272 g des in Beispiel 6 erhaltenen Produktes werden analog Beispiel 2 in alkoholischer, chinolinhaltiger Lösung über 0,068 g mit Blei vergiftetem Palladium auf Calciumcarbonat (5 % Pd) hydriert. Nach Reinigung durch HPLC mit Methanol/Wasser (85 : 15) erhält man 0,152 g der Titelverbindung.

$^1$H-NMR ($CDCl_3$):

0,63 -1,73 (m, 12 H); 2,70 -3,20 (m, 4 H); 3,90 (s, 3 H); 3,95 -4,25 (m, 1 H); 5,07 -6,63 (m, 8 H); 7,10 - 8,05 (m, 4 H).

Beispiel 8

3-(11'-Hydroxy-11'-methyl-hexadeca-1',9'-diin-1'-yl)-benzoesäuremethylester

a) 3-Methyl-3-(tetrahydro-2'-pyranyloxy)-oct-1-in

5,61 g 3-Methyl-oct-1-in-3-ol [J. Am. Chem. Soc. 76, 4446 (1954)] und 4,4 ml frisch destilliertes 3,4-Dihydropyran in 40 ml absolutem Dichlormethan werden bei 0° C unter Rühren und Überleiten von trockenem Stickstoff mit 0,019 g p-Toluolsulfonsäure-Hydrat versetzt. Nach 2 Stunden gibt man 0,80 g Kaliumcarbonat-Pulver zu und rührt dann noch für eine Stunde, wobei man die Temperatur auf 20° C ansteigen läßt. Danach filtriert man ab, wäscht den Niederschlag mit Dichlormethan, dampft das Filtrat im Vakuum ein und reinigt den Rückstand durch Säulenchromatographie mit Petrolether/Ether (5 : 1). Man erhält 7,91 g der Titelverbindung.

$^1$H-NMR ($CDCl_3$):

0,70 -2,25 (m, 20 H); 2,43 (s, 1 H); 3,23 -4,13 (m, 2 H); 4,90 -5,17 (m, 1 H).

b) 1-Brom-9-methyl-9-(tetrahydro-2'-pyranyloxy)-tetradec-7-in

In eine Lösung von 6,73 g des Produktes aus Beispiel 8a in 60 ml absolutem Tetrahydrofuran gibt man bei -78° C bis -70° C unter trockenem Stickstoff innerhalb von 45 Minuten tropfenweise 18,8 ml n-Butyllithiumlösung, rührt noch 60 Minuten im Kältebad nach und versetzt dann tropfenweise zunächst mit 13,85 ml 1,6-Dibromhexan, danach mit 15 ml absolutem Hexamethylphosphorsäuretriamid. Nach 5 Stunden wird das Kältebad entfernt. Wenn das Reaktionsgemisch sich auf 0° C erwärmt hat, versetzt man mit 60 ml gesättigter Ammoniumchloridlösung, extrahiert mit Ether und wäscht den Extrakt mehrmals mit Ammonchlorid-, dann mit gesättigter Natriumchloridlösung. Der Eindampfrückstand der über Natriumsulfat getrockneten Lösung liefert nach Säulenchromatographie mit Petrolether/Diisopropylether (15 : 1) 7,79 g der Titelverbindung.

$^1$H-NMR ($CDCl_3$):

0,67 -2,37 (m, 30 H); 3,20 -3,50 (t, 2 H); 3,23 -4,13 (m, 2 H); 4,87 -5,10 (m, 1 H).

c) 3-[11'-(Tetrahydropyranyl-2"-oxy-)-11'-methyl-hexadeca-1',9'-diin-1'-yl]-benzoesäuremethylester

Man verfährt analog Beispiel 8b und erhält aus 1,60 g 3-Ethinyl-benzoesäuremethylester, 6,25 ml n-Butyllithiumlösung sowie 1,94 g des Produktes aus Beispiel 8b unter Zusatz von 6,2 ml absolutem Hexamethylphosphorsäuretriamid nach Säulenchromatographie mit Petrolether/Ether (5 : 1) 1,61 g der Titelverbindung.
$^1$H-NMR ($CDCl_3$):

0,60 -1,97 (m, 25 H); 1,50 (s, 3 H); 2,03 -2,55 (m, 4 H); 3,20 -4,13 (m, 2 H); 3,87 (s, 3 H); 4,90 -5,13 (m, 1 H); 7,07 -8,00 (m, 4 H).

d) 3-(11'-Hydroxy-11'-methyl-hexadeca-1',9'-diin-1'-yl)-benzoesäuremethylester

Man verfährt analog Beispiel 1cIIv und erhält durch Einwirkung von 0,084 g Pyridinium-toluol-4-sulfonat auf 1,56 g des Produktes aus Beispiel 8c nach Säulenchromatographie mit n-Hexan/Ether (3 : 2) 1,07 g der Titelverbindung.
$^1$H-NMR ($CDCl_3$):

0,67 -1,93 (m, 23 H); 2,00 -2,55 (m, 4 H); 3,87 (s, 3 H); 7,07 -8,00 (m, 4 H).

Beispiel 9

3-(11'-Hydroxy-11'-methyl-hexadeca-1',9'-diin-1'-yl)-benzoesäure und deren Natriumsalz

Man verfährt analog Beispiel 3 und erhält aus 0,84 g des Produktes aus Beispiel 8 und 11 ml einer wässrigen Lösung von Lithiumhydroxid (1 Mol/l) nach Ansäuern und Aufarbeitung 0,775 g der freien Säure in Form eines Öls, das in Wasser emulgiert und unter Rühren bis pH 7 mit 0,1 n Natronlauge (ca. 21,1 ml) versetzt wird. Durch Gefriertrocknung erhält man 0,78 g des Natriumsalzes der Titelverbindung in Form weißer Kristalle.
$^1$H-NMR ($CD_3OD$):

0,67 -1,73 (m, 22 H); 2,00 -2,50 (m, 4 H); 6,97 -7,90 (m, 4 H).

Beispiel 10

4-(11'-Hydroxy-undeca-1',9'-diin-1'-yl)-benzoesäuremethylester

a) 1-Brom-9-(tetrahydro-2'-pyranyloxy)-non-7-in

Man verfährt analog Beispiel 8b und erhält aus 5,0 g 3-(Tetrahydro-2'-pyranyloxy)-1-propin, 22,31 ml n-Butyllithiumlösung sowie 16,3 ml 1,6-Dibromhexan unter Zusatz von 32,0 ml absolutem Hexamethylphosphorsäuretriamid nach Säulenchromatographie mit n-Hexan/Ether (10 : 1) 8,23 g der Titelverbindung.
$^1$H-NMR ($CDCl_3$):

1,16 -2,43 (m, 14 H); 3,20 -3,50 (m, 2 H); 3,52 -4,10 (m, 2 H); 4,61 -4,90 (m, 1 H).

b) 11-(Tetrahydro-2'-pyranyloxy)-undeca-1,9-diin

1,32 g Lithiumacetylid-Ethylendiaminkomplex werden mit 7,5 ml absolutem Dimethylsulfoxid übergossen und unter trocknem Stickstoff 30 Minuten bei Raumtemperatur gerührt. In die auf 8° C abgekühlte Mischung gibt man bei einer Temperatur von 8° C bis 9° C langsam tropfenweise 3,01 g der im Beispiel 10a erhaltenen Bromverbindung, entfernt dann das Kühlbad und rührt 1 Stunde bei Raumtemperatur nach. Man versetzt mit 5 ml gesättigter Ammoniumchloridlösung, extrahiert mehrfach mit Ether, wäscht die vereinigten Extrakte je zweimal mit gesättigten Lösungen von Ammoniumchlorid bzw. Natriumchlorid und trocknet dann über Natriumsulfat. Nach Eindampfen im Vakuum erhält man durch Säulenchromatographie [mit Petrolether/Ether (1 : 1)] des Rückstandes 2,34 g der Titelverbindung.
$^1$H-NMR ($CDCl_3$):

1,10 -1,73 (m, 14 H); 1,74 -1,99 (t, 1 H); 2,01 -2,47 (m, 4 H); 3,40 -4,10 (m, 2 H); 4,11 -4,33 (m, 2 H); 4,67 -4,87 (m, 1 H).

c) 4-[11'-(Tetrahydro-2"-pyranyloxy)-undeca-1',9'-diin-1'-yl]-benzoesäuremethylester

11,50 g des Produktes aus Beispiel 10b und 12,10 g 4-Jodbenzoesäuremethylester werden in 70 ml über Kaliumhydroxid destilliertem Triethylamin gelöst und unter Rühren mit 0,982 g Bis-(triphenylphosphin)-palladium(II)-chlorid und 0,430 g Kupfer-(I)-jodid versetzt. (DC: Petrolether/Ether -2 : 1). Nach erfolgter Umsetzung wird mit Ether verdünnt, filtriert und das Filtrat im Vakuum eingedampft. Das zurückbleibende Öl wird durch Säulenchromatographie mit Petrolether/ Ether (2 : 1) gereinigt. Man erhält 15,31 g der Titelverbindung.

$^1$H-NMR ($CDCl_3$):

1,23 -2,03 (m, 14 H); 2,04 -2,67 (m, 4 H); 3,93 (s, 3 H); 3,33 -4,07 (m, 2 H); 4,07 -4,37 (m, 2 H); 4,67 - 4,87 (m, 1 H); 7,13 -8,03 (m, 4 H).

d) 4-(11'-Hydroxy-undeca-1',9'-diin-1'-yl)-benzoesäuremethylester

Man verfährt analog Beispiel 1cIIv und erhält bei Verwendung von 1,30 g der in Beispiel 10c hergestellten Verbindung, 25 ml Ethanol und 0,086 g Pyridinium-toluol-4-sulfonat nach Säulenchromatographie mit Petrolether/Ether (1 : 1) 0,839 g der Titelverbindung.
$^1$H-NMR ($CDCl_3$):

1,16 -1,93 (m, 8 H); 1,98 -2,67 (m, 4 H); 3,94 (s, 3 H); 4,03 -4,37 (m, 2 H); 7,06 -8,01 (m, 4 H).

Beispiel 11

4-(11'-Hydroxy-undeca-1',9'-diin-1'-yl)-N-methylbenzhydroxamsäure

Zu einer Suspension von 0,179 g N-Methylhydroxylamin-Hydrochlorid in 3,5 ml absolutem Methanol gibt man unter Eiswasserkühlung tropfenweise 1,07 ml einer methanolischen Lösung von Kaliumhydroxid (3 Mol/l) und nach 90-minütigem Rühren eine Lösung von 0,321 g des in Beispiel 10 hergestellten Esters in 1 ml Methanol und entfernt dann das Kühlbad. (DC: Essigsäureethylester/Methanol -4 : 0,5). Nach Reaktionsablauf wird das Methanol im Vakuum abgedampft, der Rückstand mit 4 ml Eiswasser versetzt, mit verdünnter Salzsäure auf pH 2 -3 angesäuert und mit Essigsäureethylester extrahiert. Die vereinigten Extrakte werden mit Wasser gewaschen und über Natriumsulfat getrocknet. Nach Eindampfen wird das zurückbleibende Rohprodukt durch Säulenchromatographie mit Essigsäureethylester/Methanol (4 : 0,5) gereinigt. Man erhält 0,182 g der Titelverbindung.
$^1$H-NMR ($CDCl_3$):

1,27 -1,93 (m, 8 H); 2,01 -2,56 (m, 4 H); 3,40 (s, 3 H); 4,07 -4,33 (m, 2 H); 7,20 -7,56 (m, 4 H).

Beispiel 12

2-(11'-Hydroxy-undeca-1',9'-diin-1'-yl)-benzoesäuremethylester

a) 2-[11'-(Tetrahydro-2"-pyranyloxy)-undeca-1',9'-diin-1'-yl]-benzoesäuremethylester

Man verfährt analog Beispiel 10c und erhält aus 2,112 g 2-Jodbenzoesäuremethylester, 2,0 g des in Beispiel 10b hergestellten Produktes, 0,076 g Kupfer-(I)-jodid und 0,168 g Bis-(triphenylphosphin)-palladium-(II)-chlorid 2,52 g der Titelverbindung.
$^1$H-NMR ($CDCl_3$):

1,16 -1,94 (m, 14 H); 1,96 -2,67 (m, 4 H); 3,93 (s, 3 H); 3,33 -4,10 (m, 2 H); 4,10 -4,42 (m, 2 H); 4,60 - 4,83 (m, 1 H); 7,03 -7,83 (m, 4 H).

b) 2-(11'-Hydroxy-undeca-1',9'-diin-1'-yl)-benzoesäuremethylester

Man verfährt analog Beispiel 1cIIv und erhält aus 1,025 g des Produktes aus Beispiel 12a in 20 ml Methanol mit 0,066 g Pyridinium-toluol-4-sulfonat nach Säulenchromatographie mit Ether/Petrolether (2 : 1) 0,698 g der Titelverbindung.
$^1$H-NMR ($CDCl_3$):

1,20 -1,90 (m, 8 H); 1,23 -2,70 (m, 4 H); 3,90 (s, 3 H); 4,03 -4,37 (m, 2 H); 7,08 -7,90 (m, 4 H).

Beispiel 13

3-(11'-Hydroxy-undeca-1',9'-diin-1'-yl)-benzoesäuremethylester

a) 3-(8'-Brom-oct-1'-in-1'-yl)-benzoesäuremethylester

Man verfährt analog Beispiel 8b und erhält aus 1,60 g 3-Ethinyl-benzoesäuremethylester, 6,25 ml n-Butyllithiumlösung, 4,6 ml 1,6-Di bromhexan sowie 5 ml absolutem Hexamethylphosphorsäuretriamid nach Säulenchromatographie mit n-Hexan/Essigsäureethylester/Ether (30 : 1 : 1 ) 2,23 g der Titelverbindung.
$^1$H-NMR ($CDCl_3$):

1,33 -2,17 (m, 8 H); 2,23 -2,60 (m, 2 H); 3,27 -3,53 (t,2 H); 3,87 (s, 3 H); 7,07 -8,03 (m, 4 H).

b) 3-(8'-Jod-oct-1'-in-1'-yl)-benzoesäuremethylester

Man bringt analog Beispiel 1cIIiii 2,20 g des Produktes aus Beispiel 13a und eine Lösung von 3,06 g Natriumjodid in Aceton zur Reaktion. Nach Aufarbeitung erhält man (ohne Chromatographie) 2,39 g eines leicht gelben Öls, das für die weitere Umsetzung genügend rein ist.

$^1$H-NMR ($CDCl_3$):

1,23 -2,10 (m, 8 H); 2,23 -2,55 (m, 2 H); 3,03 -3,30 (t, 2 H); 3,87 (s, 3 H); 7,07 - 8,00 (m, 4 H).

c) 3-[11'-(Tetrahydro-2"-pyranyloxy)-undeca-1',9'-diin-1'-yl]-benzoesäuremethylester

I Man arbeitet analog Beispiel 8b und erhält aus 0,79 g 3-(Tetrahydro-2'-pyranyloxy)-prop-1-in in 17 ml absolutem Tetrahydrofuran, 3,5 ml n-butyllithiumlösung, 1,04 g 3-(8'-Jod-oct-1'-in-1'-yl)-benzoesäuremethylester in 3 ml absolutem Tetrahydrofuran sowie 5 ml absolutem Hexamethylphosphorsäuretriamid nach Säulenchromatographie mit Petrolether/Ether (5 : 1) 0,78 g der Titelverbindung.

$^1$H-NMR ($CDCl_3$):

1,30 -1,93 (m, 14 H); 2,00 -2,53 (m, 4 H); 3,20 -4,13 (m, 2 H); 3,87 (s, 3 H); 4,10 -4,27 (m, 2 H); 4,67 - 4,83 (m, 1 H); 7,05 -7,97 (m, 4 H).

Das gleiche Produkt kann man auch auf folgenden Wegen erhalten:

II Man arbeitet analog Beispiel 8b und erhält aus 0,72 g 3-Ethinyl-benzoesäuremethylester, 2,80 ml n-Butyllithiumlösung, 0,68 g des Produktes aus Beispiel 10a sowie 4 ml absolutem Hexamethylphosphorsäuretriamid nach Säulenchromatographie analog Beispiel 13cI 0,56 g des Produktes mit den gleichen spektroskopischen Resonanzdaten.

III Man bringt analog Beispiel 10c 0,579 g 11-(Tetrahydro-2'-pyranyloxy)-undeca-1,9-diin, 0,61 g 3-Jodbenzoesäuremethylester, 0,033 g Bis-(triphenylphosphin)-palladium-(II)-chlorid und 0,01 g Kupfer(I)-jodid zur Reaktion. Nach Aufarbeitung analog Beispiel 10c erhält man 0,784 g des gleichen Produktes.

d) 3-(11'-Hydroxy-undeca-1',9'-diin-1'-yl)-benzoesäuremethylester

Man verfährt analog Beispiel 1cIIv und erhält aus 2,03 g des in Beispiel 13c erhaltenen Produktes in 50 ml Ethanol mit 0,133 g Pyridinium-toluol-4-sulfonat nach Säulenchromatographie mit n-Hexan/Ether (1 : 1) 1,39 g der Titelverbindung.

$^1$H-NMR ($CDCl_3$):

1,30 -1,90 (m, 9 H); 2,07 -2,60 (m, 4 H); 3,90 (s, 3 H); 4,07 -4,37 (m, 2 H); 7,10 -8,07 (m, 4 H).

Das gleiche Produkt kann man auch gemäß Beispiel 14 herstellen.

Beispiel 14

a) Undeca-2,10-diin-1-ol

Man versetzt 3,24 g Deca-1,9-diin in 300 ml absolutem Tetrahydrofuran bei -40° C unter Rühren und Überleiten von trockenem Stickstoff innerhalb einer Stunde tropfenweise mit 59,4 ml n-Butyllithiumlösung, rührt eine Stunde nach, wobei man die Temperatur auf 0° C ansteigen läßt, gibt 6,00 g Paraformaldehyd zu und erhitzt zum Rückfluß. (DC: Petrolether/Ether -3 : 2). Nach beendeter Umsetzung wird mit gesättigter Ammoniumchloridlösung zersetzt und mehrmals mit Ether extrahiert. Die Etherphasen werden über Natriumsulfat getrocknet und im Vakuum eingedampft. Der ölige Rückstand wird durch Säulen chromatographie mit Petrolether/Ether (3 : 2) gereinigt. Dabei fallen 8,27 g der Titelverbindung in flüssiger Form an, die im Kühlschrank allmählich erstarrt und dann einen Schmelzpunkt von 25° C zeigt.

$^1$H-NMR ($CDCl_3$):

1,27 -1,80 (m, 9 H); 1,85 -2,00 (t, 1 H); 2,00 -2,40 - (m, 4 H); 4,10 -4,30 (t, 2 H).

Dieses Produkt läßt sich auch durch Abspaltung der Tetrahydropyranylgruppe aus der in Beispiel 10b hergestellten Substanz gewinnen.

b) 3-(11'-Hydroxy-undeca-1',9'-diin-1'-yl)-benzoesäuremethylester

Man bringt analog Beispiel 10c 3,29 g Undeca-2,10-diin-1-ol, 5,24 g 3-Jodbenzoesäuremethylester, 0,283 g Bis-(triphenylphosphin)-palladium-(II)-chlorid und 0,086 g Kupfer-(I)-jodid zur Reaktion. Nach Säulenchromatographie mit n-Hexan/Ether (3 : 2) erhält man 4,96 g eines Öls, dessen spektroskopische Resonanzdaten mit denen des nach Beispiel 13d gewonnenen Produktes übereinstimmen.

Beispiel 15

3-(11'-Hydroxy-undeca-1',9'-diin-1'-yl)-benzoesäure-n-hexylester

a) 3-Jodbenzoesäure-n-hexylester

1,00 g 3-Jodbenzoylchlorid wird mit 2,35 ml 1-Hexanol übergossen und drei Stunden bei Raumtemperatur gerührt. Dann gibt man die Lösung direkt auf eine mit Kieselgel 60 (0,063 -0,200 mm) beschickte Fritte und eluiert mit Petrolether/Ether - (8 : 1). Einengen des Eluats, zuletzt am Ölpumpenvakuum, liefert 1,09 g der Titelverbindung.

$^1$H-NMR ($CDCl_3$):

0,70 -2,03 (m, 11 H); 4,10 -4,40 (t, 2 H); 6,93 -8,33 (m, 4 H).

b) 3-(11'-Hydroxy-undeca-1',9'-diin-1'-yl)-benzoesäure-n-hexylester

Man bringt analog Beispiel 10c 0,164 g Undeca-2,10-diin-1-ol, 0,332 g 3-Jodbenzoesäure-n-hexylester, 0,014 g Bis-(triphenylphosphin)-palladium(II)-chlorid und 0,004 g Kupfer(I)-jodid zur Reaktion. Nach Säulenchromatographie mit n-Hexan/Ether (3 : 2) erhält man 0,298 g der Titelverbindung.

$^1$H-NMR ($CDCl_3$):

0,73 -2,05 (m, 20 H); 2,00 -2,57 (m, 4 H); 4,07 -4,40 (m, 4 H); 7,05 -8,00 (m, 4 H).

Beispiel 16

3-(11'-Hydroxy-undeca-1',9'-diin-1'-yl)-N-methyl-benzhydroxamsäure

a) 3-Jod-N-methyl-benzhydroxamsäure

8,39 g 3-Jodbenzoylchlorid werden in einem Gemisch aus 30 ml Tetrahydrofuran und 15 ml Wasser gelöst und unter Rühren bei 0° C portionsweise mit 2,22 g N-Methylhydroxylamin versetzt. Man läßt auf Raumtemperatur erwärmen und rührt noch drei Stunden. Dann wird mehrfach mit Ether extrahiert. Der Extrakt wird zweimal mit Wasser gewaschen, über Natriumsulfat getrocknet und dann im Vakuum eingedampft. Man nimmt den Rückstand in einem Gemisch aus n-Hexan und Ether (1 : 2) auf und läßt im Kühlschrank stehen. Dabei fallen 6,45 g der Titelverbindung in Form weißer Kristalle an, die bei 97° C bis 99° C - schmelzen.

$^1$H-NMR ($CDCl_3$):

3,35 (s, 3 H); 6,90 -7,87 (m, 5 H).

b) 3-(11'-Hydroxy-undeca-1',9'-diin-1'-yl)-N-methyl-benzhydroxamsäure

0,17 g Undeca-2,10-diin-1-ol, 0,287 g 3-Jod-N-methylbenzhydroxamsäure, 0,015 g Bis-(triphenylphosphin)-palladium(II)-chlorid und 0,004 g Kupfer-(I)-jodid werden analog Beispiel 10c umgesetzt. Durch Reinigung des Rohproduktes mittels HPLC unter Verwendung eines Methanol/Wasser/Tetrahydrofuran/Phosphatpuffer pH5-Gemisches (65 : 35 : 5 : 2) als Elutionsmittel erhält man 0,23 g der Titelverbindung in Form einer wachsartigen Masse.

$^1$H-NMR (DMSO-$d_6$):

1,27 -1,85 (m, 8 H); 2,00 -2,57 (m, 4 H); 3,20 (s, 3 H); 3,87 -4,13 (m, 2 H); 4,73 -5,00 (t, 1 H); 7,17 - 7,57 (m, 4 H); 9,80 (s, 1 H).

Beispiel 17

3-(11'-Hydroxy-undeca-1',9'-diin-1'-yl)-benzoesäurepiperidid

a) 3-Jod-benzoesäurepiperidid

0,90 g 3-Jodbenzoylchlorid werden unter Rühren und Eisbadkühlung in 6,8 ml Piperidin getropft. Man läßt drei Stunden bei Raumtemperatur stehen, dampft im Vakuum ein und chromatographiert den Rückstand an Kieselgel 60 (0,063 -0,200 mm) mit Petrolether/Ether (1 : 1). Man erhält so 0,97 g der Titelverbindung.

$^1$H-NMR ($CDCl_3$):

1,40 -1,80 (m, 6 H); 3,20 -3,70 (m, 4 H); 6,85 -7,73 (m, 4 H).

b) 3-(11'-Hydroxy-undeca-1',9'-diin-1'-yl)-benzoesäurepiperidid

0,12 g Undeca-2,10-diin-1-ol, 0,23 g 3-Jod-benzoesäurepiperidid, 0,011 g Bis-(triphenylphosphin)-palladium(II)-chlorid und 0,003 g Kupfer(I)-jodid werden analog Beispiel 10c zur Reaktion gebracht. Durch Säulenchromatographie des Rohproduktes mit n-Hexan/Ether (1 : 3) erhält man 0,198 g der Titelverbindung.

$^1$H-NMR ($CDCl_3$):

1,23 -1,90 (m, 15 H); 2,03 -2,57 (m, 4 H); 3,17 -3,80 (m, 4 H); 4,07 -4,30 (m, 2 H); 7,07 -7,43 (m, 4 H).

Beispiel 18

3-(11'-Hydroxy-undeca-1',9'-diin-1-yl)-benzoesäureethylester

Analog Beispiel 10c werden 0,205 g Undeca-2,10-diin-1-ol unter der katalytischen Wirkung von 0,018 g Bis-(triphenylphosphin)-palladium (II)-chlorid und 0,005 g Kupfer-(I)-jodid mit 0,345 g 3-Jodbenzoesäureethylester zur Reaktion gebracht. Nach Säulenchromatographie mit n-Hexan/Ether (1 : 1) erhält man 0,315 g der Titelverbindung.

$^1$H-NMR ($CDCl_3$):

1,10 -1,80 (m, 12 H); 2,00 -2,60 (m, 4 H); 4,03 -4,50 (m, 4 H); 7,03 -8,00 (m, 4 H).

Beispiel 19

3-(11'-Hydroxy-undeca-1',9'-diin-1'-yl)-benzoesäure-N,N-dimethylamid

a) 3-[11'-(Tetrahydro-2"-pyranyloxy)-undeca-1',9'-diin-1'-yl]-benzoesäure-N,N-dimethylamid

1,581 g 3-Jod-benzoesäure-N,N-dimethylamid (erhalten aus 3-Jodbenzoylchlorid und Dimethylamin analog Beispiel 17a) und 1,427 g des Produktes aus Beispiel 10b werden in 7,2 ml absolutem Triethylamin gelöst und mit 0,054 g Kupfer(I)-jodid sowie 0,121 g Bis-(triphenylphosphin)-palladium(II)-chlorid versetzt. (DC: Petrolether/Essigsäureethylester -3 : 4). Nach erfolgter Umsetzung wird mit Ether verdünnt, filtriert und das Filtrat im Vakuum eingedampft. Der Rückstand wird durch Säulenchromatographie mit Petrolether/Essigsäureethylester (3 : 4) gereinigt. Man erhält 1,93 g der Titelverbindung.

$^1$H-NMR ($CDCl_3$):

1,27 -1,39 (m, 14 H); 2,03 -2,60 (m, 4 H); 3,03 (s, 6 H); 3,23 -4,03 (m, 2 H); 4,03 -4,13 (m, 2 H); 4,60 - 4,90 (m, 1 H); 7,04 -7,47 (m, 4 H).

b) 3-(11'-Hydroxy-undeca-1',9'-diin-1'-yl)-benzoesäure-N,N-dimethylamid

Man verfährt analog Beispiel 1cIIv und erhält aus 0,824 g des Produktes aus Beispiel 19a in 20 ml Ethanol mit 0,055 g Pyridinium-toluol-4-sulfonat nach Säulenchromatographie mit Petrolether/Essigsäureethylester (3 : 4) 0,571 g der Titelverbindung.

$^1$H-NMR ($CDCl_3$):

1,30 -1,93 (m, 8 H); 2,04 -2,60 (m, 4 H); 3,02 (s, 6 H); 4,04 -4,37 (m, 2 H); 7,10 -7,51 (m, 4 H).

Beispiel 20

3-(11'-Hydroxy-undeca-1',9'-diin-1'-yl)-benzoesäure-n-propylester

a) 3-[11'-(Tetrahydro-2"-pyranyloxy)-undeca-1',9'-diin-1'-yl]-benzoesäure-n-propylester

Man verfährt analog Beispiel 19a und erhält aus 1,92 g 3-Jodbenzoesäure-n-propylester - (erhalten aus 3-Jodbenzoylchlorid und n-Propanol analog Beispiel 15a), 1,50 g des Produktes aus Beispiel 10b, 0,057 g Kupfer(I)-jodid, 0,126 g Bis-(triphenylphosphin)-palladium(II)-chlorid und 10 ml absolutem Triethylamin nach Säulenchromatographie mit n-Hexan/Ether (3 : 1) 2,18 g der Titelverbindung.

$^1$H-NMR ($CDCl_3$):

0,70 -1,23 (m, 3 H); 1,23 -2,56 (m, 20 H); 3,20 -4,40 (m, 6 H); 4,56 -4,93 (m, 1 H); 7,07 -8,07 (m, 4 H).

b) 3-(11'-Hydroxy-undeca-1',9'-diin-1'-yl)-benzoesäure-n-propylester

Man verfährt analog Beispiel 1cIIv und erhält aus 1,00 g des Produktes aus Beispiel 20a in 20 ml Methanol mit 0,066 g Pyridinium-toluol-4-sulfonat nach Säulenchromatographie mit Ether/Petrolether (2 : 1) 0,746 g der Titelverbindung.

$^1$H-NMR ($CDCl_3$):

0,80 -1,20 (m, 3 H); 1,27 -2,60 (m, 14 H), 3,93 -4,40 (m, 4 H); 7,10 -8,03 (m, 4 H).

Beispiel 21

3-(11'-Hydroxy-undeca-1',9'-diin-1'-yl)-benzoesäure-[N-(2'-dimethylaminoethyl)-amid] und dessen Hydrochlorid

a) 3-Jodbenzoesäure-[N-(2'-dimethylaminoethyl)-amid]

Man verfährt unter Verwendung von 6,16 ml 2-Dimethylaminoethylamin, gelöst in 25 ml absolutem Tetrahydrofuran, und 5,0 g 3-Jodbenzoylchlorid in 15 ml absolutem Tetrahydrofuran analog Beispiel 17a und erhält nach Säulenchromatographie mit Dichlormethan/Methanol (4 : 1) 4,98 g der Titelverbindung.

$^1$H-NMR ($CDCl_3$):

2,27 (s, 6 H); 2,37 -2,67 (m, 2 H); 3,20 -3,63 (m, 2 H); 6,67 -8,10 (m, 5 H).

b) 3-(11'-Hydroxy-undeca-1',9'-diin-1'-yl)-benzoesäure-[N-(2'-dimethylaminoethyl)-amid] und dessen Hydrochlorid

Man verfährt analog dem in Beispiel 10c beschriebenen Vorgehen, verwendet aber 1,51 g Undeca-2,10-diin-1-ol, 2,90 g 3-Jodbenzoesäure-[N-(2'-dimethylaminoethyl)-amid], 0,138 g Bis-(triphenylphosphin)-palladium(II)-chlorid und 0,058 g Kupfer(I)-jodid. Nach Säulenchromatographie mit Dichlormethan/Methanol (4 : 1) erhält man 2,46 g der Titelverbindung.

$^1$H-NMR ($CDCl_3$):

1,30 -1,90 (m, 8 H); 1,98 -2,73 (m, 6 H); 2,33 (s, 6 H); 3,27 -3,63 (m, 2 H); 4,13 -4,33 (m, 2 H); 6,60 - 7,80 (m, 5 H).

1 g dieser Verbindung werden in 5 ml absolutem Diethylether gelöst, tropfenweise mit 2 ml einer ca. 5 %-igen Lösung von Chlorwasserstoff in Ether versetzt, und dann wird das Reaktionsgemisch im Vakuum eingedampft. Man erhält das Hydrochlorid in Form weißer Kristalle vom Schmelzpunkt 82 -84° C.

$^1$H-NMR ($CDCl_3$):

1,33 -1,83 (m, 8 H); 2,04 -2,60 (m, 4 H); 2,90 (s, 6 H); 3,06 -3,43 (m, 2 H); 3,60 -4,10 (m, 2 H); 4,15 - 4,30 (m, 2 H); 7,23 -8,03 (m, 4 H); 8,23 -8,67 (m, 1 H).

Beispiel 22

3-(11'-Hydroxy-undeca-1',9'-diin-1'-yl)-benzonitril

1,20 g Undeca-2,10-diin-1-ol und 1,33 g 3-Brombenzonitril, gelöst in 30 ml absolutem Triethylamin, werden mit 0,105 g Bis-(triphenylphosphin)-palladium(II)-chlorid sowie 0,03 g Kupfer(I)-jodid versetzt und bei einer Badtemperatur von 70° C gerührt. (DC: Petrolether/Ether -1 : 1). Nach 6 Stunden ist die Umsetzung beendet. Man verdünnt das Reaktionsgemisch mit 50 ml Ether, filtriert und dampft das Filtrat im Vakuum ein. Der ölige Rückstand wird durch Säulenchromatographie mit Petrolether/Ether (1 : 1) gereinigt. Man erhält 1,31 g der Titelverbindung in Form eines Öls.

$^1$H-NMR ($CDCl_3$):

1,20 -1,83 (m, 8 H); 2,00 -2,57 (m, 4 H); 4,05 -4,33 (m, 2 H); 7,07 -7,65 (m, 4 H).

Beispiel 23

a) 13,42 g Deca-1,9-diin werden in 200 ml absolutem Tetrahydrofuran gelöst. In die auf -78° C bis -70° C abgekühlte Lösung gibt man innerhalb von einer Stunde tropfenweise 32 ml n-Butyllithiumlösung, rührt noch 60 Minuten im Kältebad nach und versetzt dann innerhalb von 15 Minuten die so erhaltene Lösung der Monolithiumverbindung des Deca-1,9-diins tropfenweise mit einer Lösung von 7,4 ml Cyclohexylaldehyd in 15 ml absolutem Tetrahydrofuran. Man läßt im Laufe von 5 Stunden auf 0° C erwärmen und zersetzt dann mit 50 ml gesättigter Ammoniumchloridlösung. Das Gemisch wird mit Ether extrahiert, der Extrakt mit gesättigter Natriumchloridlösung gewaschen, über Natriumsulfat getrocknet und eingedampft. Nach Säulenchromatographie des Rückstandes mit Petrolether/Ether (3 : 1) erhält man 7,22 g 1-Cyclohexyl-undeca-2,10-diin-1-ol.

$^1$H-NMR ($CDCl_3$):

0,75 -2,40 (m, 25 H); 3,90 -4,20 (m, 1 H).

b) Dieses setzt man dann analog Beispiel 10c in Gegenwart von Bis-(triphenylphosphin)-palladium-(II)-chlorid, Kupfer-(I)-jodid und Triethylamin mit den entsprechenden 3-Jodbenzoesäurederivaten um und erhält so:

i) 3-(11'-Cyclohexyl-11'-hydroxy-undeca-1',9'-diin-1'-yl)-benzoesäurepiperidid.

$^1$H-NMR ($CDCl_3$):

0,80 -2,00 (m, 26 H); 2,05 -2,57 (m, 4 H); 3,20 -3,73 (m, 4 H); 3,95 -4,17 (m, 1 H);

7,07 -7,40 (m, 4 H).

ii) 3-(11'-Cyclohexyl-11'-hydroxy-undeca-1',9'-diin-1'-yl)-benzoesäuremorpholid.

$^1$H-NMR ($CDCl_3$):

0,87 -2,07 (m, 20 H); 2,03 -2,60 (m, 4 H); 3,35 -3,87 (m, 8 H); 3,90 -4,23 (m, 1 H); 7,07 -7,43 (m, 4 H).

iii) 3-(11'-Cyclohexyl-11'-hydroxy-undeca-1',9'-diin-1'-yl)-N-methylbenzhydroxamsäure.

$^1$H-NMR ($CDCl_3$):

0,75 -2,05 (m, 20 H); 2,05 -2,60 (m, 4 H); 3,33 (s, 3 H); 3,93 -4,20 (m, 1 H); 7,13 -7,57 (m, 4 H).

iv) 3-(11'-Cyclohexyl-11'-hydroxy-undeca-1',9'-diin-1'-yl)-N-methyl-N-methoxy-benzoesäureamid.

$^1$H-NMR ($CDCl_3$):

0,75 -1,90 (m, 20 H); 1,95 -2,50 (m, 4 H); 3,27 (s, 3 H); 3,47 (s, 3 H); 3,87 -4,13 (m, 1 H); 6,93 -7,53 (m, 4 H).

v) 3-(11'-Cyclohexyl-11'-hydroxy-undeca-1',9'-diin-1'-yl)-benzoesäure-(N$^4$-methyl)-piperazid.

$^1$H-NMR ($CDCl_3$):

0,90 -2,03 (m, 20 H); 2,05 -2,53 (m, 11 H); 3,30 -3,73 (m, 4 H); 3,87 -4,15 (m, 1 H); 7,05 -7,37 (m, 4 H).

vi) 3-(11'-Cyclohexyl-11'-hydroxy-undeca-1',9'-diin-1'-yl)-benzoesäurepyrrolidid.

$^1$H-NMR ($CDCl_3$):

0,90 -2,50 (m, 28 H); 3,15 -3,70 (m, 4 H); 3,90 -4,15 (m, 1 H); 7,00 -7,37 (m, 4 H).

Beispiel 24

Man verfährt analog Beispielen 15 bis 19 und erhält aus den entsprechenden Ausgangsmaterialien:

a) 3-(11'-Hydroxy-undeca-1',9'-diin-1'-yl)-benzoesäuremorpholid.

$^1$H-NMR ($CDCl_3$):

1,33 -1,80 (m, 9 H); 2,03 -2,55 (m, 4 H); 3,37 -3,80 (m, 8 H); 4,03 -4,27 (m, 2 H); 7,05 -7,40 (m, 4 H).

b) 3-(11'-Hydroxy-undeca-1',9'-diin-1'-yl)-N-methyl-N-methoxy-benzoesäureamid.

$^1$H-NMR ($CDCl_3$):

1,33 -1,85 (m, 9 H); 2,00 -2,60 (m, 4 H); 3,27 (s, 3 H); 3,47 (s, 3 H); 4,00 -4,30 (m, 2 H); 6,93 -7,60 (m, 4 H.

c) 3-(11'-Hydroxy-undeca-1',9'-diin-1'-yl)-benzoesäure-(N$^4$-methyl)-piperazid und dessen Hydrochlorid.

$^1$H-NMR ($CDCl_3$):

1,33 -1,90 (m, 9 H); 2,05 -2,60 (m, 11 H); 3,37 -3,80 (m, 4 H); 4,10 -4,25 (m, 2 H); 7,05 -7,37 (m, 4 H).

$^1$H-NMR ($CDCl_3$) (Hydrochlorid):

1,35 -1,90 (m, 9 H); 2,03 -2,50 (m, 4 H); 2,60 -4,50 (m, 13 H); 7,10 -7,40 (m, 4 H).

d) 3-(11'-Hydroxy-undeca-1',9'-diin-1'-yl)-benzoesäurepyrrolidid.

$^1$H-NMR ($CDCl_3$):

1,33 -2,50 (m, 17 H); 3,15 -3,70 (m, 4 H); 4,07 -4,23 (m, 2 H); 7,10 -7,47 (m, 4 H).

e) N-[3-(11'-Hydroxy-undeca-1',9'-diin-1'-yl)-benzoyl]-hexamethylenimin.

$^1$H-NMR ($CDCl_3$):

1,33 -1,95 (m, 17 H); 2,00 -2,50 (m, 4 H); 3,10 -3,70 (m, 4 H); 4,05 -4,20 (m, 2 H); 6,95 -7,30 (m, 4 H).

f) 3-(11'-Hydroxy-undeca-1',9'-diin-1'-yl)-benzoesäure-n-pentylamid.

$^1$H-NMR ($CDCl_3$):

0,63 -2,53 (m, 21 H); 3,07 -3,53 (m, 2 H); 3,93 -4,13 (m, 2 H); 5,60 -6,11 (m, 1 H); 6,83

-7,53 (m, 4 H).

g) 3-(11'-Hydroxy-undeca-1',9'-diin-1'-yl)-benzoesäure-[N,N-di-(n-propyl)]-amid.

$^1$H-NMR ($CDCl_3$):

0,41 -2,63 (m, 22 H); 2,73 -3,60 (m, 4 H); 3,93 -4,27 (m, 2 H); 6,83 -7,40 (m, 4 H).

h) 3-(11'-Hydroxy-undeca-1',9'-diin-1'-yl)-benzoesäure-N-(2'-hydroxyethyl)-amid.

$^1$H-NMR ($CDCl_3$):

1,13 -2,56 (m, 12 H); 3,20 -3,83 (m, 4 H); 3,90 -4,33 (m, 2 H); 6,20 -6,77 (m, 1 H); 6,85 -7,67 (m, 4 H).

i) 3-(11'-Hydroxy-undeca-1',9'-diin-1'-yl)-benzoesäure-[N,N-di-(2'-hydroxyethyl)]-amid.

$^1$H-NMR ($CDCl_3$)

1,16 -2,60 (m, 12 H); 2,87 -4,20 (m, 10 H); 6,87 -7,33 (m, 4 H).

k) 3-(11'-Hydroxy-undeca-1',9'-diin-1'-yl)-benzoesäure-[2'-(N,N-dimethylamino)-ethyl]-ester und dessen Hydrochlorid.

$^1$H-NMR ($CDCl_3$):

1,23 -2,87 (m, 14 H); 2,33 (s, 6 H); 3,95 -4,53 (m, 4 H); 6,93 -8,11 (m, 4 H).

$^1$H-NMR ($CDCl_3$) (Hydrochlorid):

1,06 -2,53 (m, 12 H); 2,77 -2,87 (d, 6 H); 3,03 -3,53 (m, 2 H); 3,95 -4,20 (m, 2 H); 4,47 -4,87 (m, 2 H); 6,87 -7,87 (m, 4 H).

l) 3-(11'-Hydroxy-undeca-1',9'-diin-1'-yl)-benzoesäure-(N-methyl-N-acetoxy)-amid.

$^1$H-NMR ($CDCl_3$):

1,35 -1,80 (m, 9 H); 1,93 (s, 3 H); 1,90 -2,53 (m, 4 H); 3,25 (s, 3 H); 3,93 -4,23 (m, 2 H); 6,95 -7,40 (m, 4 H).

m) 3-(11'-Hydroxy-undeca-1',9'-diin-1'-yl)-benzoesäure-n-propylamid.

$^1$H-NMR ($CDCl_3$):

0,70 -2,53 (m, 17 H); 3,11 -3,51 (m, 2 H); 3,92 -4,20 (m, 2 H); 5,51 -6,13 (m, 1 H); 6,90 -7,61 (m, 4 H).

n) 3-(11'-Hydroxy-undeca-1',9'-diin-1'-yl)-benzoesäure-[N,N-di-(isopropyl)]-amid.

$^1$H-NMR ($CDCl_3$):

1,16 -2,51 (m, 24 H); 3,37 -3,73 (m, 2 H); 3,93 -4,17 (m, 2 H); 6,83 -7,27 (m, 4 H).

Beispiel 25

a) Man verfährt wie in Beispiel 23a und erhält so aus der Monolithiumverbindung des Deca-1,9-diins und Benzaldehyd 1-Phenyl-undeca-2,10-diin-1-ol, das bei Umsetzung unter den in Beispiel 10c beschriebenen Bedingungen mit 3-Jodbenzoesäuremethylester den 3-(11'-Hydroxy-11'-phenyl-undeca-1',9'-diin-1'-yl)-benzoesäuremethylester liefert.

$^1$H-NMR ($CDCl_3$):

1,33 -1,90 (m, 8 H); 1,97 -2,60 (m, 5 H); 3,83 (s, 3 H); 5,17 -5,43 (m, 1 H); 6,95 -7,95 (m, 9 H).

b) Analog Beispiel 25a erhält man unter Verwendung von Anisaldehyd anstelle von Benzaldehyd den 3-[11'-Hydroxy-11'-(4"-methoxyphenyl)-undeca-1',9'-diin-1'-yl]-benzoesäuremethylester.

$^1$H-NMR ($CDCl_3$):

1,30 -1,75 (m, 8 H); 1,90 -2,50 (m, 5 H); 3,70 (s, 3 H); 3,83 (s, 3 H); 6,57 -7,90 (m, 8 H).

c) Unter Verwendung von Veratrumaldehyd erhält man in analoger Weise den 3-[11'-Hydroxy-11'-(3",4"-dimethoxyphenyl)-undeca-1',9'-diin-1'-yl]-benzoesäuremethylester.

$^1$H-NMR ($CDCl_3$):

1,30 -1,77 (m, 8 H); 1,95 -2,50 (m, 5 H); 3,83 (s, 3 H); 3,86 (s, 6 H); 5,15 -5,40 (m, 1 H); 6,53 -7,90 (m, 7 H).

d) Verwendet man beim Vorgehen gemäß Beispiel 25a statt Benzaldehyd den Phenoxyacetaldehyd, so erhält man den 3-(11'-Hydroxy-12'-phenoxy-dodeca-1',9'-diin-1'-yl)-benzoesäuremethylester.

$^1$H-NMR ($CDCl_3$):

1,16 -1,87 (m, 8 H); 1,95 -2,56 (m, 4 H); 3,77 -4,10 (m, 2 H); 3,83 (s, 3 H); 4,37 -4,73 (m, 1 H); 6,43 -7,87 (m, 9 H).

e) Man arbeitet analog Beispiel 25a, verwendet aber Phenylacetaldehyd und in der zweiten Stufe statt 3-Jod-benzoesäure-methylester den entsprechenden n-Propylester und erhält so den 3-(11'-Hydroxy-12'-phenyl-dodeca-1',9'-diin-1'-yl)-benzoesäure-n-propylester.

$^1$H-NMR ($CDCl_3$):

0,73 -2,53 (m, 17 H); 2,83 -2,93 (d, 2 H); 3,93 -4,56 (m, 3 H); 6,83 -7,93 (m, 9 H).

Beispiel 26

a) Ausgehend von 3-Jodbenzoesäure-n-propylester stellt man analog Beispiel 23b den 3-(Deca-1',9'-diin-1'-yl)-benzoesäure-n-propylester her, löst diesen in Triethylamin und gibt zunächst eine äquimolare Menge $\beta$-Methoxypropionylchlorid, dann katalytische Mengen von Bis(triphenylphosphin)-palladium-(II)-chlorid und Kupfer-(I)-jodid zu. Man erhält so den 3-(13'-Methoxy-11'-oxo-trideca-1',9'-diin-1'-yl)-benzoesäure-n-propylester, der durch Reduktion in der im Referenzbeispiel B beschriebenen Weise in den 3-(11'-Hydroxy-13'-methoxy-trideca-1',9'-diin-1'-yl)-benzoesäure-n-propylester übergeführt wird.

$^1$H-NMR ($CDCl_3$):

0,77 -2,47 (m, 17 H); 3,27 (s, 3 H); 3,33 -3,73 (m, 2 H); 3,93 -4,53 (m, 5 H); 6,87 -7,91 (m, 4 H).

b) Geht man bei dem Verfahren des Beispiels 26a von 3-Jodbenzoesäuremethylester aus und verwendet statt des $\beta$-Methoxypropionylchlorids das Methoxyacetylchlorid, so erhält man den 3-(11'-Hydroxy-12'-methoxy-dodeca-1',9'-diin-1'-yl)-benzoesäuremethylester.

$^1$H-NMR ($CDCl_3$):

1,13 -2,60 (m, 12 H); 3,16 -3,53 (m, 5 H); 3,83 (s, 3 H); 4,16 -4,53 (m, 1 H); 6,87 -8,87 (m, 4 H).

Beispiel 27

a) Man löst 1,0 g des Produktes aus Beispiel 24f in einem Gemisch aus 10 ml absolutem Tetrahydrofuran und 1,13ml Pyridin und gibt dann unter Eiswasserkühlung tropfenweise 1,06 ml Essigsäureanhydrid zu. Anschließend läßt man auf Raumtemperatur erwärmen. Nach 20 Stunden wird im Vakuum eingedampft und der Rückstand durch Säulenchromatographie mit Ether/Petrolether (2 : 1) gereinigt. Man erhält so 0,968 g 3-(11'-Acetoxy-undeca-1',9'-diin-1'-yl)-benzoesäure-n-pentylamid in Form weißer Kristalle, die bei 52° C schmelzen.

$^1$H-NMR ($CDCl_3$):

0,67 -1,93 (m, 17 H); 2,06 (s, 3 H); 1,98 -2,67 (m, 4 H); 3,11 -3,56 (m, 2 H); 4,43 -4,67 (m, 2 H); 5,80 -6,27 (m, 1 H); 6,93 -7,67 (m, 4 H).

b) Man verfährt wie in Beispiel 27a, geht aber von den Produkten der Beispiel 20, 24g bzw. 241 aus und erhält so folgende Verbindungen:

i) 3-(11'-Acetoxy-undeca-1',9'-diin-1'-yl)-benzoesäure-n-propylester.

$^1$H-NMR ($CDCl_3$):

0,73 -2,56 (m, 17 H); 1,99 (s, 3 H); 3,90 - 4,27 (m, 2 H); 4,30 -4,63 (m, 2 H); 6,92 - 7,87 (m, 4 H).

ii) 3-(11'-Acetoxy-undeca-1',9'-diin-1'-yl)-benzoesäure-[N,N-di-(n-propyl)]-amid.

$^1$H-NMR ($CDCl_3$):

0,43 -2,51 (m, 22 H); 2,09 (s, 3 H); 2,73 - 3,47 (m, 4 H); 4,33 -4,59 (m, 2 H); 6,83 - 7,27 (m, 4 H).

iii) 3-(11'-Acetoxy-undeca-1',9'-diin-1'-yl)-benzoesäure-(N-methyl-N-acetoxy)-amid.

$^1H$-NMR ($CDCl_3$):

1,30 -1,75 (m, 8 H); 1,97 (s, 3 H); 2,03 (s, 3 H); 2,0 -2,5 (m, 4 H); 3,33 (s, 3 H); 4,5 - 4,65 (m, 2 H); 7,13 -7,47 (m, 4 H).

**Ansprüche**

1) Neue Benzoesäurederivate der allgemeinen Formel

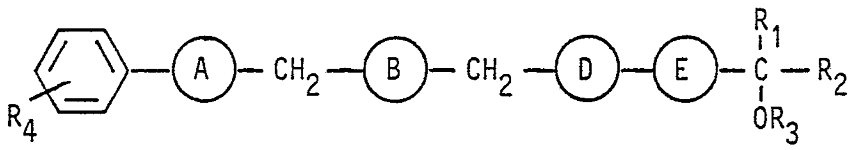

I

worin

A und E für -C≡C-und B und D für-$CH_2$-$CH_2$-stehen oder A, B und D jeweils für -C≡C-oder cis-CH = CH-und E für trans-CH = CH-stehen,

$R_1$ Wasserstoff, Methyl oder Ethyl ist,

$R_2$ Wasserstoff oder

einen geradkettigen Alkylrest mit 1 bis 6 Kohlenstoffatomen oder

eine 5-bis 7-gliedrige Cycloalkylgruppe, vorzugsweise die Cyclohexylgruppe, oder

eine Gruppe der Formel -$(CH_2)_m$-O-$R_5$, worin m für eine der Zahlen 1, 2 oder 3 und $R_5$ für Methyl oder Ethyl steht, oder

eine Gruppe der Formel

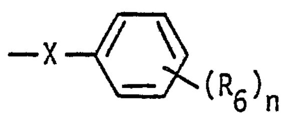

worin X für eine einfache Bindung, eine -$CH_2$-Gruppe oder für eine -$CH_2O$-Gruppe steht und $R_6$ ein Wasserstoff-, Fluor-oder Chloratom oder eine Methyl-, Methoxy-oder Trifluormethylgruppe bedeutet und n für eine der Zahlen 1 oder 2 steht,

bedeutet,

$R_3$ Wasserstoff oder ein Acetyl-oder Propionylrest ist

und

$R_4$ für eine Gruppe der Formel -$COOR_7$, worin $R_7$ ein Wasserstoffatom, ein pharmazeutisch verträgliches, insbesondere ein einwertiges Kation, ein geradkettiger oder verzweigter Alkylrest mit 1 bis 6 Kohlenstoffatomen oder die Gruppe -$(CH_2)_2$-N[-$(CH_2)_p$-$CH_3]_2$ ist, in der p für Null oder eine der Zahlen 1 bis 3 steht, sowie pharmazeutisch verträgliche Salze dieser basischen Ester mit Säuren, oder

für eine Gruppe der Formel

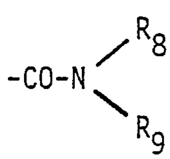

worin $R_8$ und $R_9$ gleich oder verschieden sind und für Wasserstoff, einen geradkettigen oder verzweigten Alkylrest mit 1 bis 6 Kohlenstoffatomen oder für 2-Hydroxyethyl stehen oder einer dieser Reste eine Hydroxylgruppe, der andere Wasserstoff bedeutet,

oder $R_8$ und $R_9$ zusammengenommen die Gruppe -$(CH_2)_q$-darstellen, in der q für eine der Zahlen 4, 5 oder 6 steht, oder

für eine Gruppe der Formel

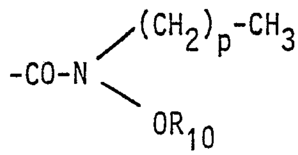

worin $R_{10}$ für Wasserstoff, $-(CH_2)_p-CH_3$, Carboxymethyl, Acetyl oder Propionyl steht und p jeweils die gleiche Bedeutung wie oben hat, oder

für eine Gruppe der Formel $-CO-NH-(CH_2)_r-N(CH_3)_2$,

worin r für eine der Zahlen 2 oder 3 steht, und deren pharmazeutisch verträgliche Salze mit Säuren, oder

für eine Gruppe der Formel

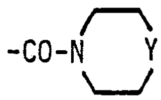

in der Y ein Sauerstoffatom oder die Gruppe

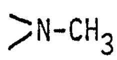

bedeutet und gegebenenfalls deren pharmazeutisch verträgliche Salze mit Säuren,

oder für einen Nitrilrest

steht.

2) Neue Benzoesäurederivate der Formel

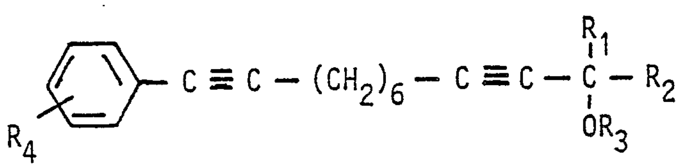

worin $R_1$ bis $R_4$ die gleiche Bedeutung wie in Anspruch 1 haben.

3) Neue Benzoesäurederivate der Formel

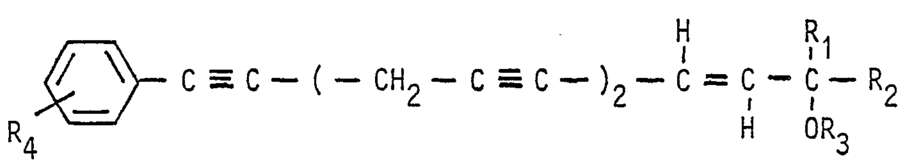 I"

worin $R_1$ bis $R_4$ die gleiche Bedeutung wie in Anspruch 1 haben.

4) Neue Benzoesäurederivate der Formel

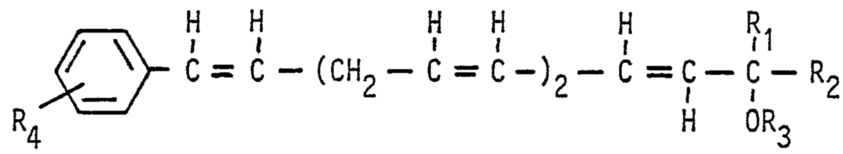

I'''

worin $R_1$ bis $R_4$ die gleiche Bedeutung wie in Anspruch 1 haben.

5) Neue Benzoesäurederivate der Formel

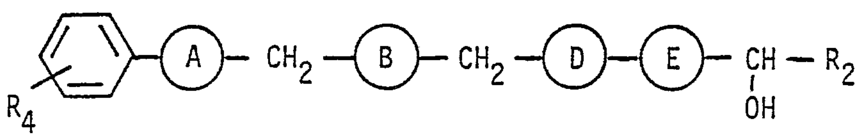

worin $R_2$, $R_4$, A, B, D und E die gleiche Bedeutung wie in Anspruch 1 haben.

6) Neue Benzoesäurederivate der Formel

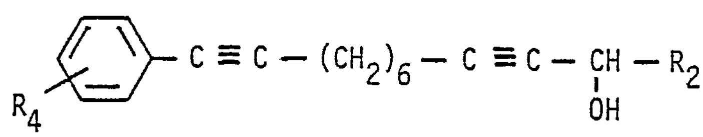

worin $R_2$ und $R_4$ die gleiche Bedeutung wie in Anspruch 1 haben.

7) Neue Benzoesäurederivate gemäß Ansprüchen 1 bis 6, in denen $R_2$ Wasserstoff, einen Alkylrest mit 5 oder 6 Kohlenstoffatomen oder einen Cyclohexylrest bedeutet.

8) Neue Benzoesäurederivate gemäß Ansprüchen 1 bis 7, in denen der Rest $R_4$ für eine Carboxylgruppe, gegebenenfalls in Form des Natrium-oder Kaliumsalzes, oder für eine Gruppe der Formeln -COO-$(CH_2)_p$-$CH_3$,

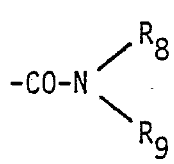

oder

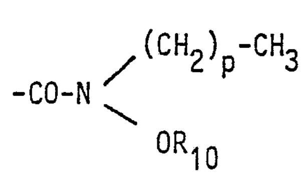

in denen $R_8$, $R_9$, $R_{10}$ und p die gleiche Bedeutung wie in Anspruch 1 haben,

steht.

9) Neue Benzoesäurederivate gemäß Ansprüchen 1 bis 8, in denen der Rest $R_4$ für die Gruppe der Formel

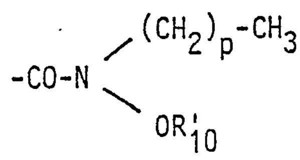

worin $R'_{10}$ Wasserstoff, $-(CH_2)_p-CH_3$ oder Acetyl bedeutet und p die gleiche Bedeutung wie oben hat,

steht.

10) Neue Benzoesäurederivate gemäß Ansprüchen 1 bis 9, in denen $R_4$ für eine der Gruppen

$-CO-NH(OH)$, $-CO-NH(OCH_3)$ oder 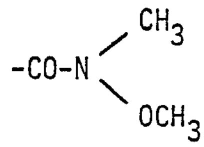 steht.

11) Arzneimittel, dadurch gekennzeichnet, daß es als Wirkstoff wenigstens eines der Benzoesäurederivate entsprechend Ansprüchen 1 bis 10 enthält.

12) Arzneimittel gemäß Anspruch 11, dadurch gekennzeichnet, daß es als Wirkstoff pro Einzeldosis 0,01 bis 50 mg eines Benzoesäurederivates entsprechend Ansprüchen 1 bis 10 enthält.

13) Arzneimittel gemäß Ansprüchen 11 und 12, dadurch gekennzeichnet, daß es zur parenteralen Applikation geeignet ist und 0,01 bis 10 mg eines Benzoesäurederivates entsprechend Ansprüchen 1 bis 10 enthält.

14) Arzneimittel gemäß Anspruch 13 zur parenteralen Applikation, dadurch gekennzeichnet, daß es in einem flüssigen, sterilen Träger 0,01 bis 10 mg eines Benzoesäurederivates entsprechend Ansprüchen 1 bis 10 in gelöster oder suspendierter Form enthält.

15) Arzneimittel gemäß Ansprüchen 11 und 12, dadurch gekennzeichnet, daß es zur oralen Applikation geeignet ist und 0,1 bis 50 mg eines Benzoesäurederivates entsprechend Ansprüchen 1 bis 10 enthält.

16) Arzneimittel gemäß Anspruch 15 zur oralen Applikation, dadurch gekennzeichnet, daß es in Form von Tabletten, Dragees oder Kapseln, gegebenenfalls mit verzögerter Wirkstofffreigabe, vorliegt.

17) Arzneimittel gemäß Anspruch 11 zur intranasalen, oralen oder peroralen Verabreichung eines Benzoesäurederivates entsprechend Ansprüchen 1 bis 10 in Spray-Form.

18) Arzneimittel gemäß Anspruch 11 zur perkutanen Applikation, bestehend aus einem auf die Haut aufzubringenden, ein Benzoesäurederivat entsprechend Ansprüchen 10 -10 in gelöster Form, vorzugsweise unter Zusatz von die Hautpenetration fördernden Mitteln, enthaltendem Reservoir.

19) Verfahren zur Herstellung der Arzneimittel gemäß Ansprüchen 11 bis 18, dadurch gekennzeichnet, daß man die Benzoesäurederivate entsprechend Ansprüchen 1 bis 10 in üblicher Weise mit Trägermaterialien, Lösungsmitteln bzw. Verdünnungsstoffen, sowie gegebenenfalls Bindemitteln, Tablettensprengstoffen und anderen üblichen Hilfsstoffen verarbeitet und aus dem erhaltenen Gemisch die Einzeldosierungsformen fertigt.

20) Verfahren zur Herstellung von neuen Benzoesäurederivaten der Formel I aus Anspruch 1, dadurch gekennzeichnet, daß man

1) bei Temperaturen von etwa -80° bis +50° C in einem aprotischen, insbesondere in einem dipolaren aprotischen Lösungsmittel, vorzugsweise in Gegenwart katalytischer Mengen eines Kupfersalzes

a) wenn A für die Gruppe -C≡C-, stehen soll, eine Verbindung der Formel

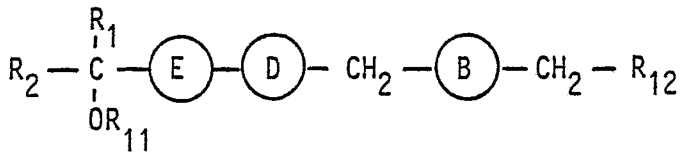

IIIa

in der B, D, E, $R_1$ und $R_2$ die gleiche Bedeutung wie in Formel I haben, $R_{11}$ eine unter milden Bedingungen abspaltbare Schutzgruppe darstellt und $R_{12}$ ein Brom-oder Jodatom oder eine Toluolsulfonyloxygruppe bedeutet,

mit einer Verbindung der Formel

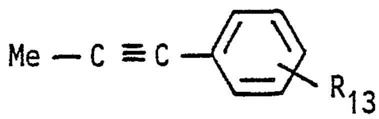

IVa

worin Me für ein Lithiumatom oder einen der Reste BrMg-oder JMg-steht und $R_{13}$ die gleiche Bedeutung wie $R_4$ mit der Maßgabe hat, daß darin $R_7$ nicht für ein Kation stehen kann und die in $R_4$ gegebenenfalls vorhandenen basischen Gruppen nicht in Salzform vorliegen können,

umsetzt, oder

b) wenn E und A für -C≡C-stehen sollen, eine Verbindung der Formel

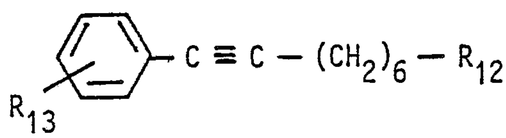

IIIb

worin $R_{12}$ und $R_{13}$ die gleiche Bedeutung wie oben haben,

mit einer Verbindung der Formel

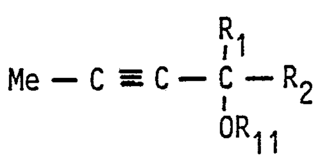

IVb

worin $R_1$, $R_2$, $R_{11}$, $R_{12}$, $R_{13}$ und Me die gleiche Bedeutung wie oben haben,

umsetzt,

aus der so erhaltenen Verbindung der Formel

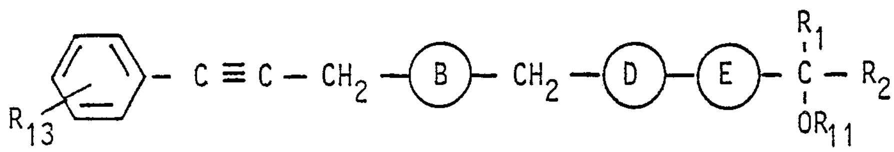

II

in der B, D, E, $R_1$, $R_2$, $R_{11}$ und $R_{13}$ die gleiche Bedeutung wie oben haben

in an sich bekannter Weise die Schutzgruppe $R_{11}$ abspaltet und -gegebenenfalls nach Einführung des Restes $R_3$, wenn dieser von Wasserstoff verschieden sein soll -so eine Verbindung der Formel

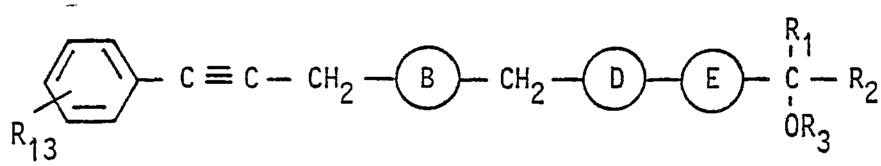

Ia

worin $R_1$ bis $R_3$, $R_{13}$, B, D und E die gleiche Bedeutung wie oben haben,

gewinnt, oder

2) eine Verbindung der Formel Ia, in der E für -C≡C-sowie B und D für $-CH_2-CH_2-$stehen, herstellt, indem man eine Verbindung der Formel

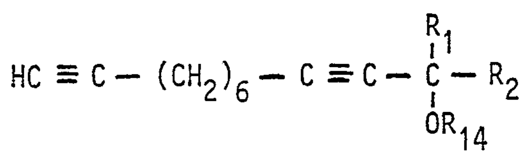

V

in der $R_1$ und $R_2$ die gleiche Bedeutung wie oben haben und $R_{14}$ für ein Wasserstoffatom steht oder die gleiche Bedeutung wie $R_{11}$ hat

mit einer Verbindung der Formel

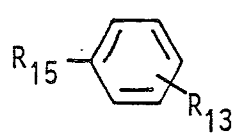

VI

worin $R_{13}$ die gleiche Bedeutung wie oben hat und $R_{15}$ für ein Brom-oder Jodatom steht,

in Gegenwart eines bei etwa -10° C bis +80° C flüssigen sekundären oder tertiären Amins und katalytischer Mengen eines komplexen Palladiumktalysators sowie gegebenenfalls von Kupfer-(I)-jodid bei Temperaturen von etwa 0° C bis 75° C zu einer Verbindung der Formel

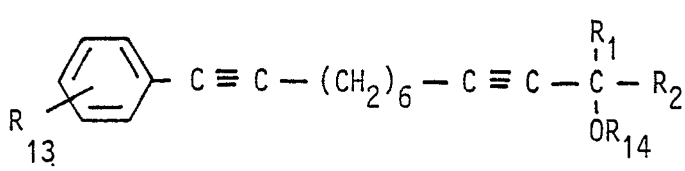

IIa

worin $R_1$, $R_2$, $R_{13}$ und $R_{14}$ die gleiche Bedeutung wie oben haben,

umsetzt und diese durch Abspaltung des Restes $R_{14}$, wenn dieser nicht für Wasserstoff steht, und gegebenenfalls Einführung eines von Wasserstoff verschiedenen Restes $R_3$ in die Verbindung der Formel Ia überführt, oder

3) eine Verbindung der Formel

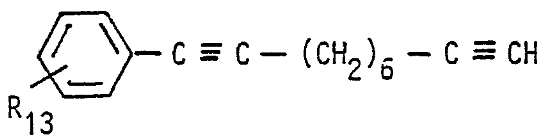

VII

worin $R_{13}$ die gleiche Bedeutung wie oben hat

in Gegenwart eines tertiären Amins under den vorstehend bei 2) für die Reaktion zwischen den Verbindungen der Formeln V und VI beschriebenen Bedingungen mit einer Verbindung der Formel

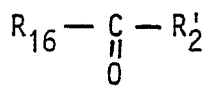

VIII

worin $R_2'$ mit Ausnahme von Wasserstoff die gleiche Bedeutung wie $R_2$ hat und $R_{16}$ für ein Chlor-, Brom-oder Jodatom steht

zur Reaktion bringt und die so erhaltene Verbindung der Formel

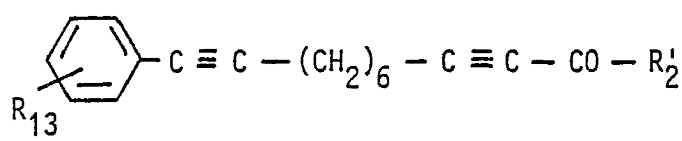

IX

worin $R_2'$ und $R_{13}$ die gleiche Bedeutung wie oben haben,

durch Reduktion mit einem Metallborhydrid oder durch Umsetzung mit einer Verbindung der Formel $R_1'$ -Me, worin Me die gleiche Bedeutung wie oben hat und $R_1'$ mit Ausnahme von Wasserstoff die gleiche Bedeutung wie $R_1$ hat, gegebenenfalls gefolgt durch Einführung eines von Wasserstoff verschiedenen Restes $R_3$, in die entsprechende Verbindung der Formel Ia, in der $R_2$ von Wasserstoff verschieden ist, überführt,

und aus der nach 1), 2) oder 3) erhaltenen Verbindung der Formel Ia durch Überführen des Restes $R_{13}$ in den Rest $R_4$ sowie gegebenenfalls (außer wenn A und E für -C≡C-stehen) durch katalytische Hydrierung einer oder mehrerer der vorliegenden Dreifachbindungen zu Doppelbindungen die Verbindungen der Formel I herstellt.

21) Verfahren gemäß Anspruch 20, dadurch gekennzeichnet, daß $R_{11}$ bzw. (sofern dieser Rest von Wasserstoff verschieden ist) $R_{14}$ eine Tetrahydropyranyl-2-, eine tert-Butyldimethylsilyl-oder eine tert-Butyldiphenylsilylgruppe ist.

22) Verfahren gemäß Ansprüchen 20 und 21, dadurch gekennzeichnet, daß bei der Verfahrensweise 1) bei etwa -80° C bis 0° C gearbeitet wird, wenn in den Formeln IVa bzw. IVb der Rest Me für ein Lithiumatom steht und die Reaktion bei etwa +5° C bis +25° C durchgeführt wird, wenn Me einen der Reste BrMg-oder JMg-bedeutet.

23) Verfahren gemäß Ansprüchen 20 und 21, dadurch gekennzeichnet, daß bei den Verfahrensweisen 2) und 3) als komplexer Palladiumkatalysator Bis-(triphenylphosphin)-palladium-(II)-chlorid oder -acetat oder Tetrakis-(triphenylphosphin)-palladium eingesetzt wird und in Gegenwart eines Trialkylamins mit 2 oder 3 Kohlenstoffatomen in jedem der Alkylreste gearbeitet wird.

24) Verfahren gemäß Anspruch 20, dadurch gekennzeichnet, daß man für die Katalytische Hydrierung von in den Gruppen A, B oder D vorliegenden Dreifachbindungen zu Doppelbindungen mit Blei vergiftete Palladiumkatalysatoren einsetzt und in Gegenwart zumindest katalytischer Mengen von Chinolin oder Pyridin arbeitet.

25) Verfahren gemäß Anspruch 20, dadurch gekennzeichnet, daß man zur Herstellung von Verbindungen der Formel I, in denen $R_4$ für eine Gruppe der Formeln

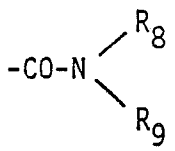 oder 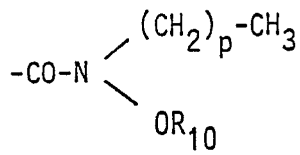

in denen $R_8$ bis $R_{10}$ und p die gleiche Bedeutung wie in Anspruch 1 haben,

steht, eine Aminoverbindung der Formeln

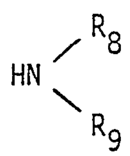 oder 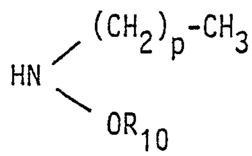

worin $R_8$ bis $R_{10}$ und p die gleiche Bedeutung wie oben haben,

a) mit einer Verbindung der Formeln Ia bzw. I, in der $R_{13}$ bzw. $R_4$ eine veresterte Carboxylgruppe ist, umsetzt, oder

b) mit einer Verbindung der Formeln Ia bzw. I, in der $R_{13}$ bzw. $R_4$ eine freie Carboxylgruppe darstellt, in Gegenwart wasserabspaltender Mittel oder nach intermediärer Bildung eines reaktiven funktionellen Derivates des Carbonsäurerestes zur Reaktion bringt.

Patentansprüche für den Vertragsstaat: AT

1) Verfahren zur Herstellung von neuen Benzoesäurederivaten der allgemeinen Formel

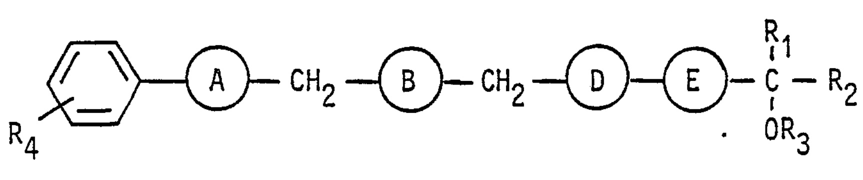 I

worin

A und E für -C≡C-und B und D für $-CH_2-CH_2-$stehen oder A, B und D jeweils für -C≡C-oder cis-CH=CH-und E für trans-CH=CH-stehen,

$R_1$ Wasserstoff, Methyl oder Ethyl ist,

$R_2$ Wasserstoff oder

einen geradkettigen Alkylrest mit 1 bis 6 Kohlenstoffatomen oder

eine 5-bis 7-gliedrige Cycloalkylgruppe, vorzugsweise die Cyclohexylgruppe, oder

eine Gruppe der Formel $-(CH_2)_m-O-R_5$, worin m für

eine der Zahlen 1, 2 oder 3 und $R_5$ für Methyl oder Ethyl steht, oder

eine Gruppe der Formel

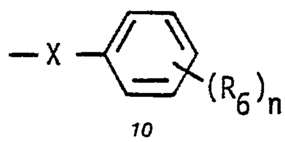

worin X für eine einfache Bindung, eine $-CH_2$-Gruppe oder für eine $-CH_2O$-Gruppe steht und $R_6$ ein Wasserstoff-, Fluor-oder Chloratom oder eine Methyl-, Methoxy-oder Trifluormethylgruppe bedeutet und n für eine der Zahlen 1 oder 2 steht,

bedeutet,

$R_3$ Wasserstoff oder ein Acetyl-oder Propionylrest ist

und

$R_4$ für eine Gruppe der Formel $-COOR_7$, worin $R_7$ ein Wasserstoffatom, ein pharmazeutisch verträgliches, insbesondere ein einwertiges Kation, ein geradkettiger oder verzweigter Alkylrest mit 1 bis 6 Kohlenstoffatomen oder die Gruppe $-(CH_2)_2-N[-(CH_2)_p-CH_3]_2$ ist, in der p für Null oder eine der Zahlen 1 bis 3 steht, sowie pharmazeutisch verträgliche Salze dieser basischen Ester mit Säuren, oder

für eine Gruppe der Formel

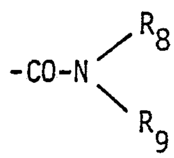

worin $R_8$ und $R_9$ gleich oder verschieden sind und für Wasserstoff, einen geradkettigen oder verzweigten Alkylrest mit 1 bis 6 Kohlenstoffatomen oder für 2-Hydroxyethyl stehen oder einer dieser Reste eine Hydroxylgruppe, der andere Wasserstoff bedeutet,

oder $R_8$ und $R_9$ zusammengenommen die Gruppe $-(CH_2)_q$-darstellen, in der q für eine der Zahlen 4, 5 oder 6 steht, oder

für eine Gruppe der Formel

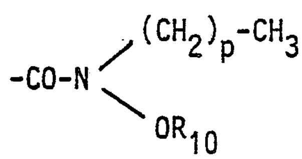

worin $R_{10}$ für Wasserstoff, $-(CH_2)_p-CH_3$, Carboxymethyl, Acetyl oder Propionyl steht und p jeweils die gleiche Bedeutung wie oben hat, oder

für eine Gruppe der Formel $-CO-NH-(CH_2)_r-N(CH_3)_2$,

worin r für eine der Zahlen 2 oder 3 steht, und deren pharmazeutisch verträgliche Salze mit Säuren, oder

für eine Gruppe der Formel

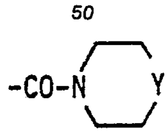

in der Y ein Sauerstoffatom oder die Gruppe

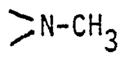

bedeutet und gegebenenfalls deren pharmazeutisch verträgliche Salze mit Säuren,

oder für einen Nitrilrest

steht, dadurch gekennzeichnet, daß man

1) bei Temperaturen von etwa -80° bis +50° C in einem aprotischen, insbesondere in einem dipolaren aprotischen Lösungsmittel, vorzugsweise in Gegenwart katalytischer Mengen eines Kupfersalzes

a) wenn A für die Gruppe -C≡C-stehen soll, eine Verbindung der Formel

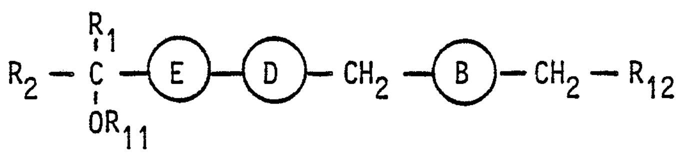

IIIa

in der B, D, E, $R_1$ und $R_2$ die gleiche Bedeutung wie in Formel I haben, $R_{11}$ eine unter milden Bedingungen abspaltbare Schutzgruppe darstellt und $R_{12}$ ein Brom-oder Jodatom oder eine Toluolsulfonyloxygruppe bedeutet,

mit einer Verbindung der Formel

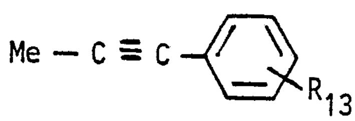

IVa

worin Me für ein Lithiumatom oder einen der Reste BrMg-oder IMg-steht und $R_{13}$ die gleiche Bedeutung wie $R_4$ mit der Maßgabe hat, daß darin $R_7$ nicht für ein Kation stehen kann und die in $R_4$ gegebenenfalls vorhandenen basischen Gruppen nicht in Salzform vorliegen können,

umsetzt, oder

b) wenn E und A für -C≡C-stehen sollen, eine Verbindung der Formel

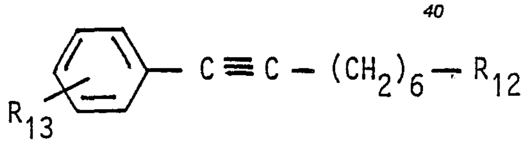

IIIb

worin $R_{12}$ und $R_{13}$ die gleiche Bedeutung wie oben haben,

mit einer Verbindung der Formel

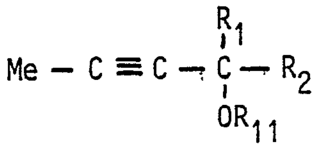

IVb

worin $R_1$, $R_2$, $R_{11}$, und Me die gleiche Bedeutung wie oben haben

umsetzt,

aus der so erhaltenen Verbindung der Formel

II

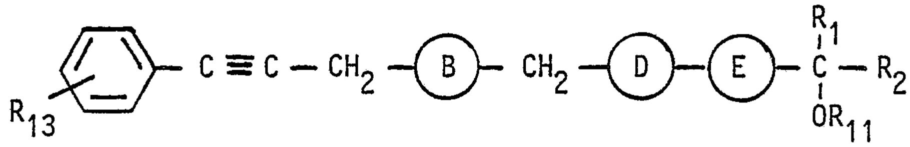

in der B, D, E, $R_1$, $R_2$, $R_{11}$ und $R_{13}$ die gleiche Bedeutung wie oben haben

in an sich bekannter Weise die Schutzgruppe $R_{11}$ abspaltet und -gegebenenfalls nach Einführung des Restes $R_3$, wenn dieser von Wasserstoff verschieden sein soll -so eine Verbindung der Formel

Ia

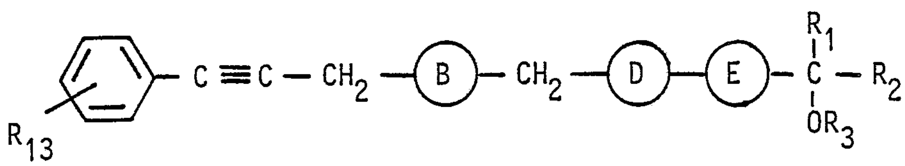

worin $R_1$ bis $R_3$, $R_{13}$, B, D und E die gleiche Bedeutung wie oben haben,

gewinnt, oder

2) eine Verbindung der Formel Ia, in der A und E für -C≡C-und B sowie D für $-CH_2-CH_2-$stehen, herstellt, indem man eine Verbindung der Formel

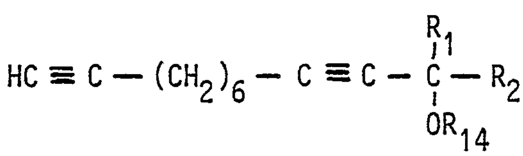

V

in der $R_1$ und $R_2$ die gleiche Bedeutung wie oben haben und $R_{14}$ für ein Wasserstoffatom steht oder die gleiche Bedeutung wie $R_{11}$ hat

mit einer Verbindung der Formel

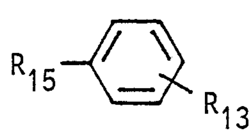

VI

worin $R_{13}$ die gleiche Bedeutung wie oben hat und $R_{15}$ für ein Brom-oder Jodatom steht,

in Gegenwart eines bei etwa -10° C bis +80° C flüssigen sekundären oder tertiären Amins und katalytischer Mengen eines komplexen Palladiumkatalysators sowie gegebenenfalls von Kupfer-(I)-jodid bei Temperaturen von etwa 0° C bis 75° C zu einer Verbindung der Formel

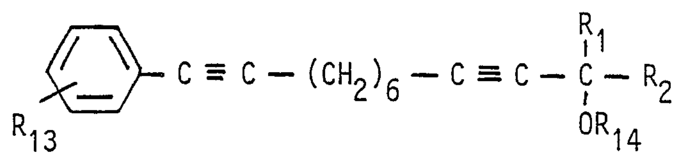

IIa

worin $R_1$, $R_2$, $R_{13}$ und $R_{14}$ die gleiche Bedeutung wie oben haben,

umsetzt und diese durch Abspaltung des Restes $R_{14}$, wenn dieser nicht für Wasserstoff steht, und gegebenenfalls Einführung eines von Wasserstoff verschiedenen Restes $R_3$ in die Verbindung der Formel Ia überführt, oder

3) eine Verbindung der Formel

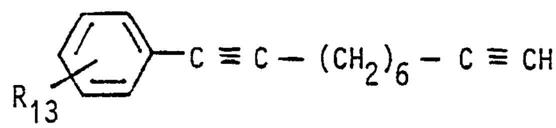

VII

worin $R_{13}$ die gleiche Bedeutung wie oben hat

in Gegenwart eines tertiären Amins unter den vorstehend bei 2) für die Reaktion zwischen der Verbindungen der Formeln V und VI beschriebenen Bedingungen mit einer Verbindung der Formel

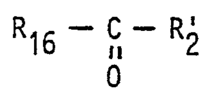

VIII

worin $R'_2$ mit Ausnahme von Wasserstoff die gleiche Bedeutung wie $R_2$ hat und $R_{16}$ für ein Chlor-, Brom-oder Jodatom steht

zur Reaktion bringt und die so erhaltene Verbindung der Formel

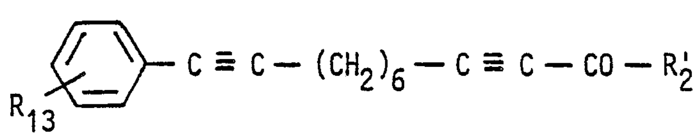

IX

worin $R'_2$ und $R_{13}$ die gleiche Bedeutung wie oben haben,

durch Reduktion mit einem Metallborhydrid oder durch Umsetzung mit einer Verbindung der Formel $R'_1$ -Me, worin Me die gleiche Bedeutung wie oben hat und $R'_1$ mit Ausnahme von Wasserstoff die gleiche Bedeutung wie $R_1$ hat, gegebenenfalls gefolgt durch Einführung eines von Wasserstoff verschiedenen Restes $R_3$, in die entsprechende Verbindung der Formel Ia, in der $R_2$ von Wasserstoff verschieden ist, überführt,

und aus der nach 1), 2) oder 3) erhaltenen Verbindung der Formel Ia durch Überführen des Restes $R_{13}$ in den Rest $R_4$ sowie gegebenenfalls (außer wenn A und E für -C≡C-stehen) durch

katalytische Hydrierung einer oder mehrerer der vorliegenden Dreifachbindungen zu Doppelbindungen die Verbindungen der Formel I herstellt.

2) Verfahren gemäß Anspruch 1, dadurch gekennzeichnet, daß $R_{11}$ bzw. (sofern dieser Rest von Wasserstoff verschieden ist) $R_{14}$ eine Tetrahydropyranyl-2-, eine tert-Butyldimethylsilyl-oder eine tert-Butyldiphenylsilylgruppe ist.

3) Verfahren gemäß Ansprüchen 1 und 2, dadurch gekennzeichnet, daß bei der Verfahrensweise 1) bei etwa -80° C bis 0° C gearbeitet wird, wenn in den Formeln IVa bzw. IVb der Rest Me für ein Lithiumatom steht und die Reaktion bei etwa +5° C bis +25° C durchgeführt wird, wenn Me einen der Reste BrMg-oder IMg-bedeutet.

4) Verfahren gemäß Ansprüchen 1 und 2, dadurch gekennzeichnet, daß bei den Verfahrensweisen 2) und 3) als komplexer Palladiumkatalysator Bis-(triphenylphosphin)-palladium-(II)-chlorid oder -acetat oder Tetrakis-(triphenylphosphin)-palladium eingesetzt werden und in Gegenwart eines Trialkylamins mit 2 oder 3 Kohlenstoffatomen in jedem der Alkylreste gearbeitet wird.

5) Verfahren gemäß Anspruch 1, dadurch gekennzeichnet, daß man für die katalytische Hydrierung von in den Gruppen A, B oder D vorliegenden Dreifachbindungen zu Doppelbindungen mit Blei vergiftete Palladiumkatalysatoren einsetzt und in Gegenwart zumindest katalytischer Mengen von Chinol in oder Pyridin arbeitet.

6) Verfahren gemäß Anspruch 1, dadurch gekennzeichnet, daß man zur Herstellung von Verbindungen der Formel I, in denen $R_4$ für eine Gruppe der Formeln

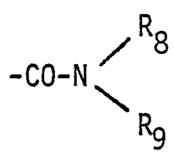 oder 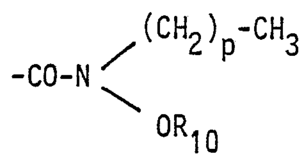

in denen $R_8$ bis $R_{10}$ und p die gleiche Bedeutung wie in Anspruch 1 haben,

steht, eine Aminoverbindung der Formeln

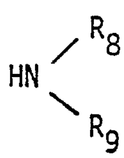 oder 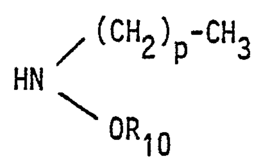

worin $R_8$ bis $R_{10}$ und p die gleiche Bedeutung wie oben haben,

a) mit einer Verbindung der Formeln Ia bzw. I, in der $R_{13}$ bzw. $R_4$ eine veresterte Carboxylgruppe ist, umsetzt, oder

b) mit einer Verbindung der Formeln Ia bzw. I, in der $R_{13}$ bzw. $R_4$ eine freie Carboxylgruppe darstellt, in Gegenwart wasserabspaltender Mittel oder nach intermediärer Bildung eines reaktiven funktionellen Derivates des Carbonsäurerestes zur Reaktion bringt.